(19) Europäisches Patentamt
European Patent Office
Office européen des brevets

(11) Veröffentlichungsnummer: **0 243 313**
**B1**

(12) # EUROPÄISCHE PATENTSCHRIFT

(45) Veröffentlichungstag der Patentschrift: **16.05.90**

(51) Int. Cl.$^5$: **C07C 323/11**, C07C 317/06, C07C 317/18, A01N 35/06, A01N 41/10

(21) Anmeldenummer: **87810238.3**

(22) Anmeldetag: **15.04.87**

(54) **Acyl-cyclohexandione und deren Oximäther mit herbizider und das Pflanzenwachstum regulierender Wirkung.**

(30) Priorität: **24.04.86 CH 1664/86**

(43) Veröffentlichungstag der Anmeldung:
**28.10.87 Patentblatt 87/44**

(45) Bekanntmachung des Hinweises auf die Patenterteilung:
**16.05.90 Patentblatt 90/20**

(84) Benannte Vertragsstaaten:
**AT BE CH DE ES FR GB GR IT LI LU NL SE**

(56) Entgegenhaltungen:
**EP-A- 0 090 262**
**EP-A- 0 099 534**
**DE-A- 3 123 018**
**DE-B- 2 822 304**

**Agricultural and Biological Chemistry, Band 37, Nr. 1, Jänner 1973, Agricultural Chemical Society of Japan Takayuki Oritani et al. "Syntheses of Pentadienoic Acids Structurally Related to Abscisic Acid" Seiten 261-268**

(73) Patentinhaber: **CIBA-GEIGY AG, Klybeckstrasse 141, CH-4002 Basel(CH)**

(72) Erfinder: **Tobler, Hans, Dr., Baselmattweg 157, CH-4123 Allschwil(CH)**

**EP 0 243 313 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-Acyl-1,3-cyclohexandione und deren Oximäther mit herbizider und das Pflanzenwachstum regulierender Wirkung, Verfahren zur Herstellung der 2-Acyl-1,3-cyclohexandione und deren Oximäther, Mittel welche diese Derivate enthalten sowie die Verwendung dieser Derivate oder sie enthaltender Mittel zur Unkrautbekämpfung und zur Regulierung des Pflanzenwachstums.

Die neuen 2-Acyl-1,3-cyclohexandione und deren Oximäther entsprechen der Formel I

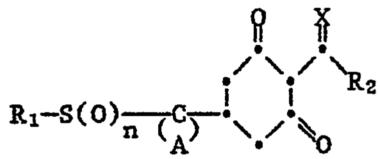

(I)

worin A eine 2-7-gliedrige Alkylenbrücke, eine 3-7-gliedrige Alkenylenbrücke, welche ein- oder mehrfach ungesättigt sein kann,
n Null, eins oder zwei
$R_1$ $C_1$-$C_4$-Alkyl oder Benzyl
$R_2$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl, wobei der Phenylring durch Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,
X Sauerstoff oder ein Rest $=NOR_3$ und
$R_3$ $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl
bedeuten.

Die Erfindung umfasst ebenfalls die durch die verschiedenen Endformen gekennzeichneten Isomeren, Enantiomeren und Diastereomeren der Formel I sowie die Salze dieser Verbindungen mit Metallen und Stickstoffbasen.

In diesen Definitionen sind unter dem Begriff Alkyl selbst oder als Bestandteil eines anderen Substituenten, wie Alkoxy, Alkylthio, Halogenalkyl, Halogenalkylthio, sowohl geradkettige wie verzweigte Reste, z.B. Methyl, Aethyl, Propyl, Isopropyl, Butyl, Isobutyl, sec.Butyl, tert.Butyl sowie alle stereoisomeren Formen der höheren Homologen. Ebenso werden unter Alkenyl und Alkinyl geradkettige wie verzweigte Reste verstanden sowie deren cis- und trans-Formen, z.B. Allyl, Methallyl, Butenyl, Methyl- und Dimethylbutenyl, Propinyl, Butinyl, Methylbutinyl, Dimethylbutinyl.

Cycloalkylreste als Substituenten $R_2$ oder welche durch das Kohlenstoffatom und die Alkylenbrücke A gebildet werden umfassen Cyclopropyl, Cyclobutyl, Cyclopentyl, Cyclohexyl, Cycloheptyl sowie Cyclooctyl. Durch das Kohlenstoffatom und die Alkenylenbrücke A gebildete Cycloalkenylenreste können ein- oder mehrfach ungesättigt sein. Beispiele dafür sind Cyclopropenyl, Cyclobutenyl, Cyclopentenyl, Cyclopentadienyl, Cyclohexenyl, Cyclohexadienyl, Cycloheptenyl, Cycloheptadienyl, Cycloheptatrienyl, Cyclooctenyl, Cyclooctadienyl und Cyclooctatrienyl. Diese Reste können mit bis zu 5 Methylgruppen substituiert werden.

Unter Halogen sind Fluor-, Chlor-, Brom oder Jodatome zu verstehen.

Die 2-Acyl-1,3-cyclohexandione und ihre Oximäther der Formel I zeichnen sich durch gute herbizide und Pflanzenwuchs regulierende Wirkung aus. Unter den Verbindunge, die durch ihre Wirkung besonders aufgefallen sind, befinden sich folgende Gruppen:
Die Acylcyclohexandione der Formel I, in denen
A eine 2-7 gliedrige Alkylenbrücke,
n Null, eins oder zwei,
$R_1$ $C_1$-$C_4$-Alkyl oder Benzyl,
$R_2$ $C_1$-$C_6$-Alkyl, unsubstituiert oder substituiert durch Halogen, $C_1$-$C_4$-Alkoxy oder $C_1$-$C_4$-Alkylthio; $C_3$-$C_6$-Cycloalkyl; Phenyl, Benzyl oder Phenyläthyl, wobei der Phenylring durch Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann und
X Sauerstoff bedeuten, unter welchen die Verbindungen
5-(1-Methylthiocyclobutan-1-yl)-2-(2,4-dichlorobenzoyl)-cyclohexan-1,3-dion,
5-(1-Methylthiocyclobutan-1-yl)-2-n-butyryl-cyclohexan-1,3-dion
5-(1-Methylthiocyclobutan-1-yl)-2-cyclopropylcarbonyl-cyclohexan-1,3-dion,
5-(1-Methylthiocyclobutan-1-yl)-2-(2,3-dichlorobenzoyl)-cyclohexan1,3-dion,
5-(1-Methylsulfonylcyclobutan-1-yl)-2-n-butyryl-cyclohexan-1,3-dion,
5-(1-Methylthiocyclopropan-1-yl)-2-propionyl-cyclohexan-1,3-dion,
5-(1-Aethylthiocyclopropan-1-yl)-2-propionyl-cyclohexan-1,3-dion und
5-(1-Methylthiocyclopropan-1-yl)-2-n-butyryl-cyclohexan-1,3-dion.

Ferner sind die Oximäther der Acylcyclohexandione der Formel I aufgefallen, in denen
A eine 2-7 gliedrige Alkylenbrücke,
n Null, eins oder zwei,
$R_1$ $C_1$-$C_4$-Alkyl oder Benzyl,
$R_2$ $C_1$-$C_6$-Alkyl, unsubstituiert oder substituiert durch Halogen, $C_1$-$C_4$-Alkoxy oder $C_1$-$C_4$-Alkylthio,
X den Rest = $NOR_3$ und
$R_3$ $C_1$-$C_6$-Alkyl, $C_3$-$C_6$-Halogenalkyl, $C_3$-$C_6$-Alkenyl, $C_3$-$C_6$-Halogenalkenyl oder $C_3$-$C_6$-Alkinyl,
bedeuten, besonders die Verbindungen
5-(1-Methylthiocyclobutan-1-yl)-2-(1-äthoximino-n-butyryl)-cyclohexan-1,3-dion,
5-(1-Methylthiocyclopropan-1-yl)-2-(1-äthoximino-n-butyryl)-cyclohexan-1,3-dion,
5-(1-Methylthiocyclohexan-1-yl)-2-(1-äthoximino-n-butyryl)-cyclohexan-1,3-dion,
5-(1-Methylthiocyclobutan-1-yl)-2-(1-allyloxyimino-n-butyryl)-cyclohexan-1,3-dion und
5-(1-Methylthiocyclopentan-1-yl)-2-(1-äthoximino-n-butyryl)-cyclohexan-1,3-dion,
5-(1-Methylthiocyclopropan-1-yl)-2-[1-(trans-3-chlorallyloximino)-n-butyryl]-cyclohexan-1,3-dion,
5-(1-Methylthiocyclopropan-1-yl)-2-[1-(trans-3-chlorallyloximino)-propionyl]-cyclohexan-1,3-dion,
5-(1-Methylthiocyclopropan-1-yl)-2-[1-(cis-3-chlorallyloximino)-propionyl]-cyclohexan-1,3-dion und
5-(1-Aethylthiocyclopropan-1-yl)-2-[1-(trans-3-chlorallyloximino)-propionyl]-cyclohexan-1,3-dion.

Diese Verbindungen können auch in tautomeren Formen oder als Salze vorliegen. Insbesondere kommen Alkali- und Erdalkalimetallsalze sowie Mangan, Kupfer, Zink und Eisensalze in Betracht.

Die Herstellung der erfindungsgemässen Acyl-Cyclohexandione und deren Oximäther erfolgt in an sich bekannter Weise durch Umsetzen eines in 5-Stellung durch den Rest entsprechend substituierten 1,3-Cyclohexandions mit einem Säurechlorid oder einem Säurecyanid und gegebenenfalls weiterer Umsetzung des erhaltenen 2-Acyl-1,3-cyclohexandions mit einem Hydroxylamin.

Ein erstes Verfahren zur Herstellung der Acyl-Cyclohexandione und deren Oximäther der Formel I ist dadurch gekennzeichnet, dass man in einem inerten organischen Lösungsmittel, in Gegenwart der äquimolaren Menge einer Base, ein 1,3-Cyclohexandionderivat der Formel II

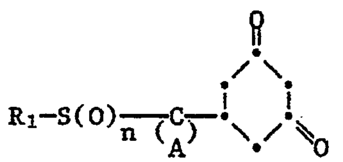

(II)

worin A, n und $R_1$ die unter der Formel I gegebene Bedeutung haben, mit einem Säurehalogenid oder Säureanhydrid der Formel III umsetzt

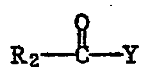

(III)

worin Y ein Halogenatom oder einen Rest

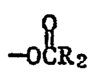

bedeutet und $R_2$ die unter der Formel I gegebene Bedeutung hat, den erhaltenen Cyclohexanon-Ester der Formel IV

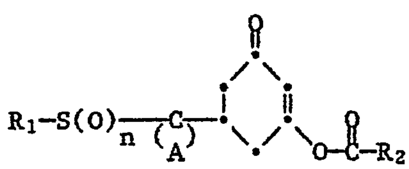

(IV)

worin
A, n, $R_1$ und $R_2$ die unter der Formel I gegebene Bedeutung haben, in einem inerten organischen Lösungsmittel in Gegenwart eines Katalysators zum 2-Acyl-1,3-cyclohexan-dion-derivat der Formel Ia umlagert,

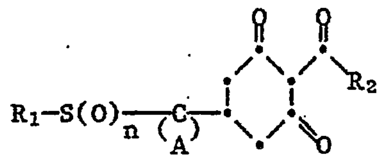

(Ia)

worin A, n, $R_1$ und $R_2$ die unter der Formel I angegebene Bedeutung haben, und dieses gewünschtenfalls in einem inerten organischen Lösungsmittel, in Gegenwart der äquimolaren Menge einer Base, mit einem Hydroxylamin • Hydrochlorid der Formel V
$H_2NOR_3 \cdot HCl$ (V)
worin $R_3$ die unter der Formel I gegebene Bedeutung hat, zum Oximäther der Formel Ib umsetzt,

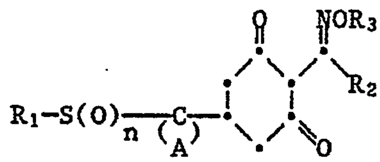

(Ib)

worin A, n, $R_1$, $R_2$ und $R_3$ die unter der Formel I gegebene Bedeutung haben.

Die Cyclohexanon-ester der Formel IV sind neue Verbindungen. Sie und ihre Herstellung sind Gegenstand dieser Erfindung.

Ein zweites direkteres Verfahren zur Herstellung der Acyl-Cyclohexandione und deren Oximäther der Formel I ist dadurch gekennnzeichnet, dass man in einem inerten organischen Lösungs- oder Verdünnungsmittel, in Gegenwart von Zinkchlorid sowie einer Stickstoffbase ein 1,3-Cyclohexandionderivat der Formel II

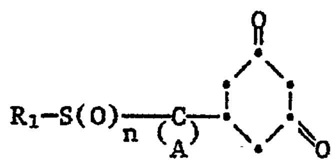

(II)

worin A, n und $R_1$ die unter der Formel I gegebene Bedeutung haben, mit einem Säurecyanid der Formel VI umsetzt

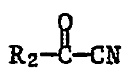

(VI)

worin $R_2$ die unter der Formel I gegebene Bedeutung hat, und das erhaltene 2-Acyl-1,3-cyclohexandionderivat der Formel Ia

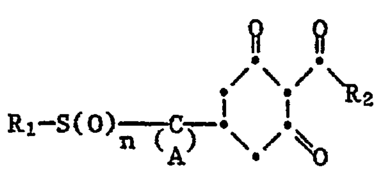

(Ia)

worin A, n, $R_1$ und $R_2$ die unter der Formel I gegebene Bedeutung haben, gewünschtenfalls in einem inerten organischen Lösungsmittel, in Gegenwart der äquimolaren Menge einer Base mit einem Hydroxylamin • Hydrochlorid der Formel V
$H_2NOR_3 \cdot HCl$ (V)
worin $R_3$ die unter der Formel I gegebene Bedeutung hat, zum Oximäther der Formel Ib umsetzt

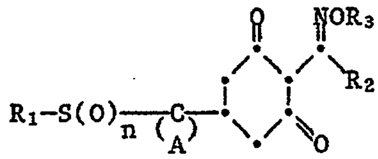

(Ib)

worin A, n, $R_1$, $R_2$ und $R_3$ di unter der Formel I gegebene Bedeutung haben.

Als inerte organische Lösungsmittel für diese Umsetzungen kommen vor allem Aromaten wie Benzol, Toluol, Halogenwasserstoffe wie Chloroform, Dichloräthan, Dichlormethan, Tetrachlorkohlenstoff, Ester wie z.B. Aethylacetat in Frage.

Die Reaktionstemperaturen liegen zwischen Raumtemperatur und dem Siedepunkt des Reaktionsgemisches. Während der Zugabe von Säurechlorid ist möglicherweise eine Kühlung des Reaktionsgefässes angezeigt.

Als Basen kommen organische wie anorganische in Betracht. Beispiele sind Pyridin, 4-Aminopyridin, 4-Dimethylaminopyridin, Kollidin, Triäthylamin, Ammonium-, Natrium-, Kalium- oder Calciumcarbonat oder die entsprechenden Bicarbonate.

Solche Umsetzungen sind bekannt. Die Umsetzung mit dem Säurehalogenid oder Säureanhydrid der Formel III ist in Tetrahedron Letters 29 (1973) 249 oder in Synthesis 1978, 925, die Umsetzung mit dem Säurecyanid der Formel V in der EP-A 90 262 beschrieben.

Die Umlagerung des Cyclohexanonesters der Formel IV zum 2-Acyl-cyclohexandionderivat der Formel Ia erfolgt z.B. durch Behandeln in einem inerten organischen Lösungsmittel in Gegenwart eines Katalysators oder in einer organischen Base als Lösungsmittel. Als Katalysatoren kommen z.B. Pyridin, 4-Aminopyridin, 4-(Dimethylamino)-pyridin, Aluminium(III)chlorid in Methylenchlorid, Collidin, Lutidin, Cyanhydrine zusammen mit Stickstoffbasen wie Triäthylamin in Frage.

Die Herstellung der als Ausgangsprodukte benötigten Cyclohexandione der Formel II erfolgt über ein mehrstufiges Verfahren.

Ein ungesättigtes Methylketon der Formel VII

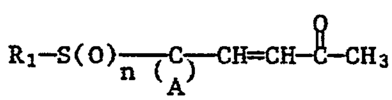

(VII)

worin A, n und $R_1$ die unter der Formel I gegebene Bedeutung haben, wird in einem absoluten, inerten organischen Lösungsmittel in Gegenwart von Alkalimetall-methylat bei Rückflusstemperatur des Lösungsmittels mit einem Malonsäurediester der Formel VIII umgesetzt

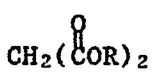

(VIII)

wobei R einen $C_1$-$C_6$-Alkylrest oder Benzyl bedeutet, zum Cyclohex-1-en-2-ol-4-on-ester der Formel IX

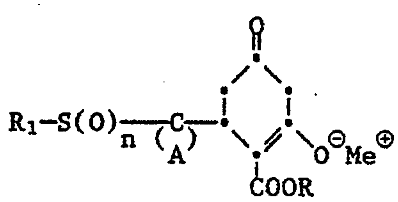

(IX)

worin A, n und $R_1$ die unter der Formel I gegebene Bedeutung hat, R einen $C_1$-$C_6$-Alkylrest oder Benzyl und $Me^{\oplus}$ ein Alkalimetallion bedeutet, diesen Ester in Gegenwart von Natron- oder Kalilauge verseift und mit Säure wäscht, das erhaltene 2,4-Cyclohexandion-säure-derivat der Formel X

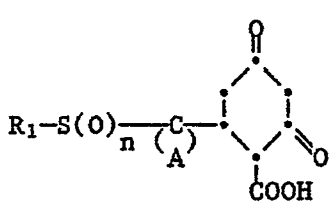

(X)

worin A, n und $R_1$ die unter der Formel I gegebene Bedeutung haben, dann in einem inerten Lösungsmittel

decarboxyliert und das als Ausgangsprodukt benötigte Cyclohexandion der Formel II

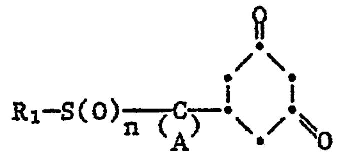

(II)

worin A, n und $R_1$ die unter der Formel I gegebene Bedeutung hat aus dem Reaktionsgemisch isoliert.

Die Umsetzung des ungesättigten Methylketons der Formel VII mit dem Malonsäurediester der Formel VIII wird in einem absoluten Lösungsmittel in Gegenwart von vorzugsweise Natrium- oder Kaliummethylat durchgeführt. Als Malonsäureester sind die Aethyl-und Methylester bevorzugt. Die Verseifung und das darauffolgende Ausfällen der Säure geschieht in wässrigem Medium. Das 2,4-Cyclohexandion-1-carbonsäure-derivat der Formel X wird dann in einem Lösungsmittel, wie z.B. Wasser, Toluol, Xylol oder in einem chlorhaltigen Lösungsmittel wie Methylenchlorid oder Chloroform verkocht bis keine Kohlensäure mehr entsteht.

Ein anderer Weg zur Herstellung der 1,3-Cyclohexandion-derivate der Formel II besteht darin, dass man einen Aldehyd der Formel XI

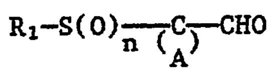

(XI)

worin A, n und $R_1$ die unter der Formel I gegebene Bedeutung haben in einem basischen Lösungsmittel mit Malonsäure zur ungesättigten Säure der Formel XII kondensiert,

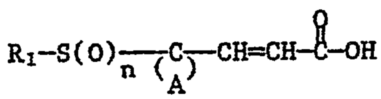

(XII)

worin A, n und $R_1$ die unter der Formel I gegebene Bedeutung haben. Diese Säure wird dann in bekannter Weise mit einem Alkanol R-OH, worin R $C_1$-$C_6$-Alkyl oder Benzyl bedeutet, verestert und der Ester der Formel XIV in einem absoluten Lösungsmittel, in Gegenwart von Natriummethylat oder Kaliummethylat mit einem Acetessigsäure-alkylester der Formel XV ringgeschlossen, entsprechend dem Formelschema:

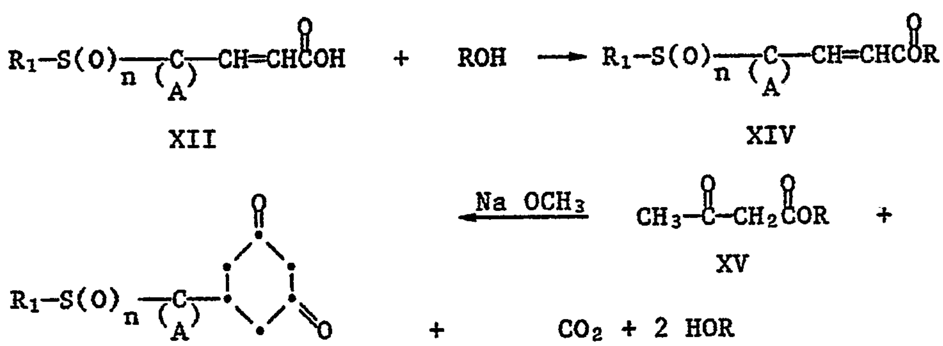


XII XIV XV II

Die Kondensation des Aldehydes der Formel XI mit Malonsäure findet entweder in einem basischen Lösungsmittel wie Pyridin, Collidin, Lutidin, oder in einem absoluten Alkanol, z.B. Aethanol oder Methanol in Gegenwart von Natrium-äthylat oder Natrium-Methylat.

Die 1,3-Cyclohexandion-derivate der Formel II sind neue Produkte und ihre Herstellung bilden einen Gegenstand dieser Erfindung.

Die als Ausgangsmaterial benötigten Methylketone der Formel VII werden durch Kondensation von Aldehyden der Formel XI mit Aceton und nachfolgender Wasserabspaltung des als Kondensationsprodukt erhaltenen β-Hydroxyketons.

Die Reaktion kann durch folgendes Schema wiedergegeben werden:

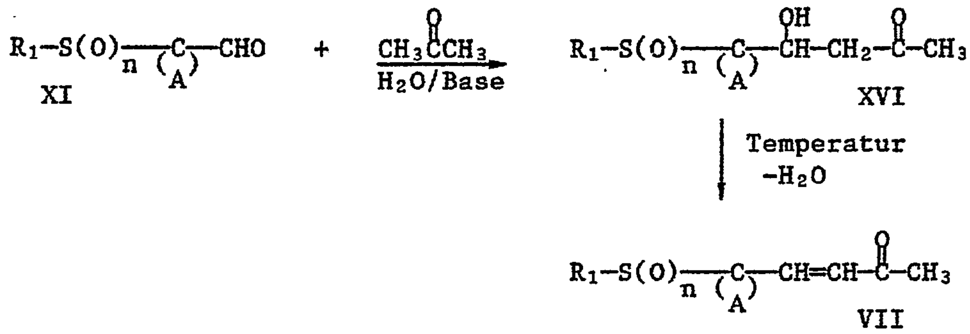


XI XVI VII

Die Kondensation findet in wässrigem Milieu in Gegenwart einer Base z.B. Natron- oder Kalilauge statt, vorteilhafterweise bei höherer Temperatur z.B. dem Siedepunkt des Reaktionsgemisches.

Die 1-Alkylthio- resp. 1-Alkylsulfinyl- oder 1-Alkylsulfonyl-cycloalkyl-carbaldehyde der Formel IX sind bekannt, ihre Herstellung ist beispielsweise in der DE-A 2,120,908 beschrieben oder in Analogie zu DE-A 2,403,236 realisierbar.

Die ungesättigten Methylketone den Formel VII sind neue Produkte. Sie und ihre Herstellung sind Gegenstand dieser Erfindung.

Das Verfahren zur Herstellung der ungesättigten Methylketone der Formel VII

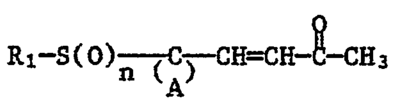

(VII)

worin A, n und $R_1$ die unter der Formel I gegebene Bedeutung haben, ist dadurch gekennzeichnet dass man einen Aldehyd der Formel XI

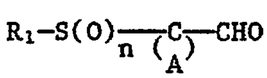

(XI)

worin A, n und $R_1$ die unter der Formel I gegebene Bedeutung haben, in basisch-wässrigem Milieu mit Aceton kondensiert, dann während mehreren Stunden am Rückfluss kocht und es anschliessend aus dem Reaktionsgemisch isoliert.

Ein ähnliches Verfahren zur Herstellung von ungesättigten Ketonen ist beispielsweise in Agr. Biol. Chem. 37 (1973) 261 beschrieben.

Die beschriebenen Herstellungsverfahren sind, einschliesslich aller Teilschnitte, ein wichtiger Bestandteil der vorliegenden Erfindung. Die neuen Wirkstoffe sind Feststoffe oder Oele die sich problemlos handhaben lassen.

Die Verbindungen der Formel I haben herbizide und das Pflanzenwachstum regulierende Wirkung, sie eignen sich z.B. zur selektiven Bekämpfung von Gräsern in Nutzpflanzenkulturen. Besonders wirksam waren die trans-3-chlorallyloximäther-Verbindungen.

Bei geringen Aufwandmengen zeichnen sich die Verbindungen der Formel I durch gute wuchshemmende und selektiv-herbizide Eigenschaften aus, die sie ausgezeichnet zum Einsatz in Kulturen von Nutzpflanzen, insbesondere in Zuckerrüben, Getreide, Baumwolle, Soja, Mais und Reis befähigen. Es werden dabei teilweise auch Unkräuter geschädigt, welchen bisher nur mit Totalherbiziden beizukommen war.

Die Verbindungen der Formel I haben überdies gute pflanzenwuchsregulierende Eigenschaften.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung aufgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanze von der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z.B. mehr oder grössere Früchte zur Ausbildung kommen.

Ertragssteigerungen können in manchen Fällen auch durch einen Eingriff in den pflanzlichen Stoffwechsel erreicht werden, ohne dass sich Aenderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen, z.B. um bei Tabakpflanzen nach der Dekapitierung die Ausbildung von Seitentrieben zu verhindern und damit das Blattwachstum zu fördern.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin könne Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden Vorraussetzungen dafür geschaffen, dass z.B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen, also die endogene Jahresrhythmik, beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z.B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosythetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbedeckungspflanzen, den sogenannten Cover crops. In tropischen und subtropischen Monokulturen, wie z.B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der

Austrocknung, Versorgung mit Stickstoff) und zur Verhinderung von Erosion (Abtragung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachsum beeinflussende Präparate sein; es können aber auch selektive Herbizide, Insektizide, Fungizide, Bakterizide, Nematizide, Molluskizide oder Gemische mehrerer dieser Präparate sein, zusammen mit gegegebenenfalls weiteren in der Formulierungtechnik üblicher Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach der Art der Wachstumsbeeinflussung. Die Werkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zuberietung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100 g bis 3 kg AS/ha, insbesondere bei 200 g bis 1000 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegegebenenfalls epoxidiertes Pflanzenöl wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Tpyen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substiutierte Ammoniumsalze von höheren Fettsäuren ($C_{10}$-$C_{22}$), wie z.B. die Na- oder K-Salze der Oel- oder Stearinsäure, oder von natürlichen Fettsäuregemischen die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkai-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkhol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro PropylenglykolEinheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Oxtylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979.

M. and J. Ash, "Encyclopedia of Surfactants" Vol. I-III, Chemical Publishing Co., Inc. New York, 1981.

H. Stache "Tensid-Taschenbuch" 2. Auflage C. Hanser Verlag, München und Wien 1981

Diese Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesonders 0,1 bis 25 %, eines Tensides.

Insbesondere setzen sich bevorzugte Formulierungen folgendermassen zusammen: (% = Gewichtsprozent)

<u>Lösungen</u>

Aktiver Wirkstoff: 5 bis 95 %, vorzugsweise 10 bis 80 %
Lösungsmittel: 95 bis 5 %, vorzugsweise 90 bis 0 %
oberflächenaktives Mittel: 1 bis 30 %, vorzugsweise 2 bis 20 %.

<u>Emulgierbare Konzentrate</u>

Aktiver Wirkstoff 10 bis 50 %, bevorzugt 10 bis 40 %
oberflächenaktives Mittel: 5 bis 30 %, vorzugsweise 10 bis 20 %
flüssiges Trägermittel: 20 bis 95 %, vorzugsweise 40 bis 80 %.

<u>Stäube</u>

Aktiver Wirkstoff: 0,5 bis 10 %, vorzugsweise 2 bis 8 %
festes Trägermaterial: 99,5 bis 90 %, vorzugsweise 98 bis 92 %.

<u>Suspensions-Konzentrate</u>

Aktiver Wirkstoff 5 bis 75 %, vorzugsweise 10 bis 50 %
Wasser: 94 bis 25 %, vorzugsweise 90 bis 30 %
oberflächenaktives Mittel: 1 bis 40 %, vorzugsweise 2 bis 30 %.

Benetzbare Pulver

Aktiver Wirkstoff: 5 bis 90 %, vorzugsweise 10 bis 80 %
und insbesondere 20 bis 60 %
oberflächenaktives Mittel: 0,5 bis 20 %, vorzugsweise 1 bis 15 %
festes Trägermaterial: 5 bis 90 %, vorzgusweise 30 bis 70 %.

Granulate

Aktiver Wirkstoff: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben, Druckangaben in Millibar (mbar).

Beispiel: 1

Herstellung von 1-(trans-But-3-en-2-on-4-yl)-1-methylthio-cyclobutan (Zwischenprodukt)

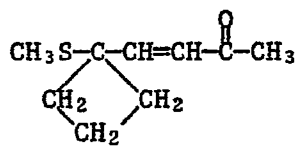

Zu einer Lösung von 39 g Cyclobutan-1-methylthio-1-carbaldehyd in 400 ml Aceton tropft man unter Rühren bei 50°C während 5 Minuten 140 ml wässrige 2N Natronlauge. Das Gemisch wird dann während 15 Stunden bei Raumtemperatur und 8 Stunden unter Rückfluss weitergerührt. Dann wird das Reaktionsgemisch eingedampft, der Rückstand in Aether aufgenommen, mit Wasser und Salzsole gewaschen, getrocknet und der Aether abgedampft. Das zurückbleibende Oel wird bei 0,013 mbar destilliert. Man erhält 41.5 g eines klaren Oeles, das bei 58°/0,013 mbar siedet und einen Gehalt an Titelprodukt von 96.5 % aufweist (Gaschromatographie).

In analoger Weise zu diesem Beispiel werden die in der Tabelle 1 aufgeführten Methylketone der Formel VII, welche als Zwischenprodukte dienen, hergestellt.

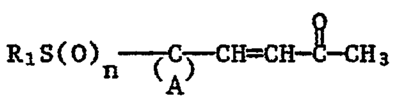

(VII)

Tabelle 1

| No . | $R_1$ | n | A | phys. Daten |
|---|---|---|---|---|
| 1.01 | $CH_3$ | 0 | $(CH_2)_2$ | Kp. 54-55°/0,013 mbar |
| 1.02 | $C_2H_5$ | 0 | $(CH_2)_2$ | Kp. 66-69°/0,013 mbar |
| 1.03 | $CH_3$ | 0 | $(CH_2)_3$ | Kp. 58°/0,013 mbar |
| 1.04 | $C_2H_5$ | 0 | $(CH_2)_3$ | Kp. 70-72°/0,05 mbar |
| 1.05 | $C_3H_7$-n | 0 | $(CH_2)_3$ | |
| 1.06 | $CH_3$ | 0 | $(CH_2)_4$ | Kp. 74°/0,013 mbar |
| 1.07 | $C_2H_5$ | 0 | $(CH_2)_4$ | |
| 1.08 | Benzyl | 0 | $(CH_3)_4$ | |
| 1.09 | $CH_3$ | 0 | $(CH_2)_5$ | Kp. 85-88°/0,013 mbar |
| 1.10 | $C_2H_5$ | 0 | $(CH_2)_5$ | Kp. 90-92°/0,013 mbar |
| 1.11 | $C_3H_7$-n | 0 | $(CH_2)_5$ | |
| 1.12 | $CH_3$ | 0 | $(CH_2)_6$ | |
| 1.13 | $C_2H_5$ | 0 | $(CH_2)_6$ | |
| 1.14 | $C_3H_7$-n | 0 | $(CH_2)_2$ | Kp. 74-79°/0,013 mbar |
| 1.15 | $CH(CH_3)_2$ | 0 | $(CH_2)_2$ | Kp. 69-72°/0,013 mbar |
| 1.16 | Benzyl | 0 | $(CH_2)_2$ | Kp. 138-141°/0,013 mbar |
| 1.17 | $CH_3$ | 1 | $(CH_2)_2$ | |
| 1.18 | $CH_3$ | 2 | $(CH_2)_2$ | |
| 1.19 | $CH_3$ | 1 | $(CH_2)_3$ | |
| 1.20 | $CH_3$ | 2 | $(CH_2)_3$ | |

Beispiel 2

Herstellung von 5-(1-Methylthio-cyclobutan-1-yl)-cyclohexan-1,3-dion (Zwischenprodukt)

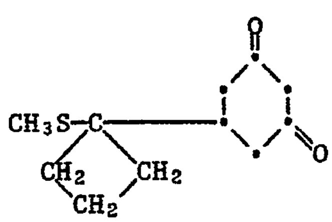

Zu einer gerührten Suspension von 33,5 g Dimethylmalonat und 47 g Natriummethylat (30.8%ig in Methanol) in 900 ml absolutem Toluol tropft man während 15 Minuten 41 g 1-(trans-But-3-en-2-on-1-yl)-1-methylthio-cyclobutan. Das breiartige Reaktionsgemisch wird dann innert 5 Stunden zum Rückfluss erhitzt.

Dabei lässt man Methanol wegdestillieren bis eine Destillationstemperatur von 110° erreicht ist. Das anfänglich schwer rührbare Reaktionsgemisch geht dabei in eine feine Suspension über. Nach dem Abkühlen dampft man zur Trockne ein und wäscht den Rückstand mit Hexan. Man erhält so 70 g Natrium Salz des 5-(1-Methylthio-cyclobutan-1-yl)-6-methoxycarbonyl-cyclohex-6-en-3-on-1-ol's der Formel

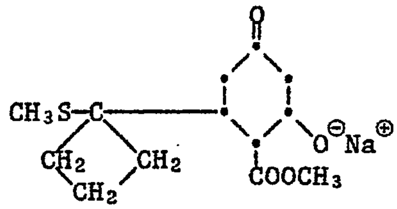

als Zwischenprodukt.

Dieses wird in 250 ml wässriger 2N Kalilauge gelöst, und während 1¼ Stunden bei 80°C gerührt. Dann lässt man abkühlen und beginnt bei 70° langsam 80 ml konzentrierte Salzsäure zutropfen. Nach dem Abkühlen fällt das Produkt kristallin aus. Man filtiert es ab, wäscht mit Wasser bis das Waschwasser neutral ist und trocknet bei 40-50°C im Exsikkator. Ausbeute 48 g. Schmelzpunkt nach Umkristallisieren aus Aethanol/Wasser 141-143°.

In analoger Weise zu diesem Beispiel werden die in der Tabelle 2 aufgeführten 1,3-Cyclohexandion-derivate, welche als Zwischenprodukte benötigt werden, erhalten.

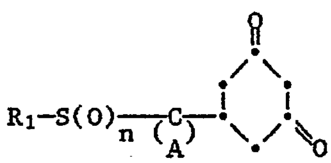

(II)

Tabelle 2

| No. | $R_1$ | n | A | phys. Daten |
|---|---|---|---|---|
| 2.01 | $CH_3$ | 0 | $(CH_2)_3$ | Smp. 141-143° |
| 2.02 | $C_2H_5$ | 0 | $(CH_2)_3$ | Smp. 92-100° |
| 2.03 | $CH_3$ | 0 | $(CH_2)_2$ | Smp. 140-145° |
| 2.04 | $C_2H_5$ | 0 | $(CH_2)_2$ | Smp. 118-121° |
| 2.05 | $C_3H_7$-n | 0 | $(CH_2)_2$ | Smp. 107-109° |
| 2.06 | $CH_3$ | 0 | $(CH_2)_4$ | Smp. 138-140° |
| 2.07 | $C_2H_5$ | 0 | $(CH_2)_4$ | |
| 2.08 | $C_2H_5$ | 1 | $(CH_2)_4$ | |
| 2.09 | $C_2H_5$ | 2 | $(CH_2)_4$ | |
| 2.10 | $CH_3$ | 0 | $(CH_2)_5$ | Smp. 138-140° |
| 2.11 | $C_2H_5$ | 0 | $(CH_2)_5$ | Smp. 99-101° |
| 2.12 | $C_3H_7$-n | 0 | $(CH_2)_5$ | |
| 2.13 | Benzyl | 0 | $(CH_2)_5$ | |
| 2.14 | $CH(CH_3)_2$ | 0 | $(CH_2)_2$ | Smp. 98-104°C |
| 2.15 | Benzyl | 0 | $(CH_2)_2$ | Smp. 163-165°C |
| 2.16 | $CH_3$ | 1 | $(CH_2)_2$ | |
| 2.17 | $CH_3$ | 2 | $(CH_2)_2$ | |
| 2.18 | $C_2H_5$ | 1 | $(CH_2)_2$ | |
| 2.19 | $C_2H_5$ | 2 | $(CH_2)_2$ | |
| 2.20 | $CH_3$ | 1 | $(CH_2)_3$ | |
| 2.21 | $CH_3$ | 2 | $(CH_2)_3$ | |

Beispiel 3

Herstellung von 5-(1-Methylthio-cyclobutan-1-yl)-3-n-butyryloxycyclohex-2-en-1-on (Zwischenprodukt)

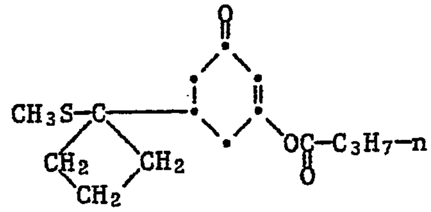

Eine Mischung von 13,8 g Cyclohexan-1,3-dion, 8,7 g Butyrylchlorid und 11,3 g Kaliumcarbonat in 250 ml Tetrahydrofuran wird während 4 Stunden bei Raumtemperatur gerührt. Dann wird das Reaktionsgemisch eingedampft, in Aether aufgenommen, zweimal mit Wasser und einmal mit Salzsole gewaschen und getrocknet. Der Aether wird abgedampft, das verbleibende Oel mit Aether-Hexan 1:2 über eine Kieselgel-Flash-Kolonne chromatographiert. Nach Eindampfen des Eluates verbleiben 10,2 g eines farblosen Oeles $n_D^{26}$ 1.275.

In analoger Weise zu diesem Beispiel werden die in der Tabelle 3 aufgeführten Cyclohexanonester der Formel IV, welche als Zwischenprodukte benötigt werden, erhalten.

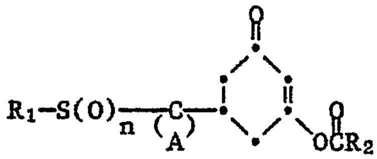

(IV)

Tabelle 3

| No. | $R_1$ | n | A | $R_2$ | phys. Daten |
|---|---|---|---|---|---|
| 3.01 | $CH_3$ | 0 | $(CH_2)_3$ | $C_3H_7$-n | $n_D^{26}$ 1.5275 |
| 3.02 | $CH_3$ | 0 | $(CH_2)_3$ | Cyclopropyl | $n_D^{35}$ 1.5478 |
| 3.03 | $C_2H_5$ | 0 | $(CH_2)_3$ | $C_3H_7$-n | $n_D^{35}$ 1.5218 |
| 3.04 | $C_2H_5$ | 0 | $(CH_2)_3$ | Cyclopropyl | |
| 3.05 | $C_2H_5$ | 0 | $(CH_2)_3$ | $C_2H_5$ | $n_D^{30}$ 1.5253 |
| 3.06 | $CH_3$ | 0 | $(CH_2)_2$ | $C_3H_7$-n | $n_D^{26}$ 1.5210 |
| 3.07 | $CH_3$ | 0 | $(CH_2)_2$ | Cyclopropyl | $n_D^{26}$ 1.5413 |
| 3.08 | $C_2H_5$ | 0 | $(CH_2)_2$ | $C_3H_7$-n | Kp. 142°/0.013 mbar |
| 3.09 | $C_2H_5$ | 0 | $(CH_2)_2$ | Cyclopropyl | $n_D^{25}$ 1.5358 |
| 3.10 | $C_2H_5$ | 0 | $(CH_2)_2$ | $C_2H_5$ | Kp. 137°/0.013 mbar |
| 3.11 | $C_2H_5$ | 0 | $(CH_2)_2$ | $CH(CH_3)_2$ | |
| 3.12 | $CH_3$ | 0 | $(CH_2)_4$ | $C_3H_7$-n | $n_D^{26}$ 1.5297 |
| 3.13 | $C_2H_5$ | 0 | $(CH_2)_4$ | $C_3H_7$-n | |
| 3.14 | $CH_3$ | 0 | $(CH_2)_4$ | Cyclopropyl | $n_D^{26}$ 1.5437 |
| 3.15 | $CH_3$ | 0 | $(CH_2)_2$ | $C_2H_5$ | $n_D^{26}$ 1.5310 |

Tabelle 3 (Fortsetzung)

| No. | $R_1$ | n | A | $R_2$ | phys. Daten |
|---|---|---|---|---|---|
| 3.16 | $C_2H_5$ | 0 | $(CH_2)_4$ | $C_2H_5$ | |
| 3.17 | $CH_3$ | 0 | $(CH_2)_5$ | $C_3H_7$-n | $n_D^{30}$ 1.5306 |
| 3.18 | $CH_3$ | 0 | $(CH_2)_5$ | Cyclopropyl | Smp. 90-91° |
| 3.19 | $C_2H_5$ | 0 | $(CH_2)_5$ | $C_3H_7$-n | $n_D^{30}$ 1.5263 |
| 3.20 | $C_2H_5$ | 0 | $(CH_2)_5$ | Cyclopropyl | |
| 3.21 | $CH_3$ | 0 | $(CH_2)_3$ | Benzyl | |
| 3.22 | $CH_3$ | 0 | $(CH_2)_3$ | $C_2H_5$ | $n_D^{26}$ 1.5356 |
| 3.23 | $C_2H_5$ | 0 | $(CH_2)_5$ | $C_2H_5$ | $n_D^{30}$ 1.5333 |
| 3.24 | $C_2H_5$ | 0 | $(CH_2)_4$ | $CH_3$ | |
| 3.25 | $CH_3$ | 0 | $(CH_2)_5$ | $C_2H_5$ | Smp. 76-77° |
| 3.26 | $CH(CH_3)_2$ | 0 | $(CH_2)_2$ | $C_2H_5$ | $n_D^{30}$ 1.5167 |
| 3.27 | $CH(CH_3)_2$ | 0 | $(CH_2)_2$ | $C_3H_7$-n | $n_D^{30}$ 1.5125 |
| 3.28 | $CH(CH_3)_2$ | 0 | $(CH_2)_2$ | Cyclopropyl | $n_D^{30}$ 1.5298 |
| 3.29 | $C_3H_7$-n | 0 | $(CH_2)_2$ | $C_2H_5$ | Kp. 140-142°/0,013 mbar |
| 3.30 | $C_3H_7$-n | 0 | $(CH_2)_2$ | $C_3H_7$-n | Kp. 153-155°/0,013 mbar |
| 3.31 | $C_3H_7$-n | 0 | $(CH_2)_2$ | Cyclopropyl | $n_D^{30}$ 1.5290 |
| 3.32 | $C_3H_7$-n | 0 | $(CH_2)_2$ | $CH(CH_3)_2$ | $n_D^{30}$ 1.5096 |
| 3.33 | $CH_3$ | 0 | $(CH_2)_2$ | $CH_3$ | $n_D^{30}$ 1.5303 |
| 3.34 | $CH_3$ | 0 | $(CH_2)_2$ | $C_4H_9$-n | |
| 3.35 | Benzyl | 0 | $(CH_2)_2$ | $C_2H_5$ | Smp. 69-71° |
| 3.36 | Benzyl | 0 | $(CH_2)_5$ | $C_3H_7$-n | $n_D^{30}$ 1.5562 |
| 3.37 | $CH_3$ | 1 | $(CH_2)_2$ | $C_2H_5$ | |
| 3.38 | $CH_3$ | 2 | $(CH_2)_2$ | $C_3H_7$-n | |
| 3.39 | $CH_3$ | 2 | $(CH_2)_2$ | $C_2H_5$ | |
| 3.40 | $CH_3$ | 1 | $(CH_2)_2$ | $C_3H_7$-n | |
| 3.41 | $CH_3$ | 1 | $(CH_2)_3$ | $C_2H_5$ | |
| 3.42 | $CH_3$ | 2 | $(CH_2)_3$ | $C_3H_7$-n | |
| 3.43 | Benzyl | 0 | $(CH_2)_2$ | Cyclopropyl | Smp. 62-64° |

Beispiel 4

Herstellung von 5-(1-Methylthiocyclobutan-1-yl)-2-n-butyryl-cyclohexan-1,3-dion

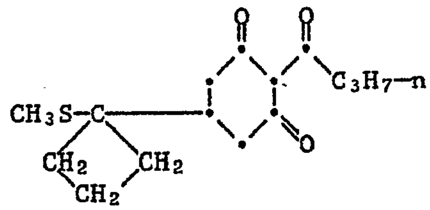

Eine Lösung von 10,0 g 5-(1-Methylthio-cyclobutan-1-yl)-3-n-butyryloxy-cyclohex-2-en-1-on (Beispiel 3) und 0,5 g 4-(N,N-Dimethylamino)-pyridin werden während 3 Tagen bei einer Temperatur von 100-110 °C gerührt. Das Reaktionsgemisch wird dann eingedampft. Das verbleibende Oel wird über eine Kieselgelkolonne welche 300 g Kieselgel und obendrauf eine 1 cm Schicht "Aluminiumoxid sauer" enthält mit Aether-Hexan 1:5 chromatographiert. Nach Verdampfen des Eluates verbleiben 8,1 g Titelprodukt als gelbes Oel $n_D^{35}$ 1.5498.

Beispiel 5

Herstellung von 5-(1-Methylthiocyclobutan-1-yl)-2-(2,4-dichlorbenzoyl-cyclohexan-1,3-dion

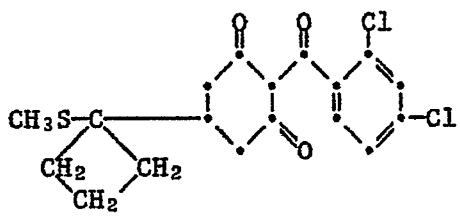

Zu einem Gemisch von 3,71 g 5-(1-Methylthiocyclobutan-1-yl)-cyclohexan-1,3-dion (Beispiel 2), 3,85 g 2,4-Dichlorbenzoylcyanid und 2,62 g Zinkchlorid in 100 ml Methylenchlorid gibt man unter Kühlen im Eisbad und Rühren tropfenweise 1,94 g Triäthylamin. Das Reaktionsgemisch lässt man dann sich auf Raumtemeperatur erwärmen und rührt während 20 Stunden weiter. Dann giesst man auf eine Mischung von Eis und konzentrierter Salzsäure 1:1, gibt Methylenchlorid dazu und trennt die organische Phase ab. Diese wird zweimal mit Wasser gewaschen, getrocknet und eingedampft. Der Rückstand wird mit Essigester/Hexan 1:1 über eine 150 g Kieselgel-Säule chromatographiert, auf die man eine 1 cm dicke Schicht von Aluminiumoxid (Alox I sauer) gegeben hat. Das Eluat wird eingedampft und der Rückstand in Aether aufgenommen. Unlöslicher Rückstand wird abfiltriert. Es verbleibt nach dem Verdampfen des Aethers ein viskoses Oel, das beim Stehen erstarrt. Man verreibt es in Hexan und erhält so 3,4 g kristallines Material. Schmelzpunkt 82-83°.

In analoger Weise zu den Beispielen 4 und 5 werden die in der Tabelle 4 aufgeführten 2-Acyl-1,3-cyclohexandione der Formel Ia hergestellt.

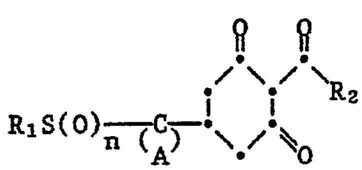

(Ia)

Tabelle 4

| No. | $R_1$ | n | A | $R_2$ | phys. Daten |
|---|---|---|---|---|---|
| 4.01 | $CH_3$ | 0 | $(CH_2)_3$ | $C_3H_7$-n | $n_D^{25}$ 1.5498 |
| 4.02 | $CH_3$ | 0 | $(CH_2)_3$ | Cyclopropyl | $n_D^{35}$ 1.5753 |
| 4.03 | $CH_3$ | 0 | $(CH_2)_3$ | 2,4-Dichlorphenyl | Smp. 82-83° |
| 4.04 | $CH_3$ | 0 | $(CH_2)_3$ | 2,3-Dichlorphenyl | Smp. 88-91° |
| 4.05 | $C_2H_5$ | 0 | $(CH_2)_3$ | $C_3H_7$-n | $n_D^{30}$ 1.5432 |
| 4.06 | $C_2H_5$ | 0 | $(CH_2)_3$ | Cyclopropyl | |
| 4.07 | $C_2H_5$ | 0 | $(CH_2)_3$ | 2,4-Dichlorphenyl | |
| 4.08 | $C_2H_5$ | 0 | $(CH_2)_3$ | 4-Chlorphenyl | |
| 4.09 | $CH_3$ | 0 | $(CH_2)_4$ | $C_3H_7$-n | $n_D^{26}$ 1.5536 |
| 4.10 | $C_2H_5$ | 0 | $(CH_2)_4$ | $C_3H_7$-n | |
| 4.11 | $C_2H_5$ | 0 | $(CH_2)_4$ | Cyclopropyl | |
| 4.12 | $CH_3$ | 0 | $(CH_2)_4$ | Cyclopropyl | Smp. 80-82° |
| 4.13 | $C_2H_5$ | 0 | $(CH_2)_4$ | 2,4-Dichlorphenyl | |
| 4.14 | $CH_3$ | 0 | $(CH_2)_4$ | 2,4-Dichlorphenyl | Smp. 89-91° |
| 4.15 | $C_2H_5$ | 0 | $(CH_2)_4$ | $C_2H_5$ | |
| 4.16 | $C_2H_5$ | 0 | $(CH_2)_4$ | $CH(CH_3)_2$ | |
| 4.17 | $C_2H_5$ | 2 | $(CH_2)_4$ | $C_3H_7$-n | |
| 4.18 | $CH_3$ | 0 | $(CH_2)_5$ | $C_3H_7$-n | $n_D^{30}$ 1.5541 |
| 4.19 | $C_2H_5$ | 0 | $(CH_2)_5$ | $C_3H_7$-n | $n_D^{30}$ 1.5483 |
| 4.20 | $C_2H_5$ | 0 | $(CH_2)_5$ | Cyclopropyl | |
| 4.21 | $CH_3$ | 0 | $(CH_2)_5$ | Cyclopropyl | $n_D^{30}$ 1.5711 |
| 4.22 | $C_2H_5$ | 0 | $(CH_2)_5$ | 2,4-Dichlorphenyl | |
| 4.23 | $C_2H_5$ | 0 | $(CH_2)_5$ | 2-Chlorphenyl | |
| 4.24 | $C_2H_5$ | 0 | $(CH_2)_5$ | $C_2H_5$ | Smp. 100-101° |
| 4.25 | $CH_3$ | 0 | $(CH_2)_3$ | $C_2H_5$ | $n_D^{26}$ 1.5566 |
| 4.26 | $CH_3$ | 0 | $(CH_2)_4$ | 4-Chlorphenyl | Smp. 108-110° |
| 4.27 | $CH_3$ | 0 | $(CH_2)_2$ | Cyclopropyl | Smp. 71-73° |
| 4.28 | $CH_3$ | 0 | $(CH_2)_2$ | $C_3H_7$-n | Smp. 61-62° |
| 4.29 | $CH_3$ | 0 | $(CH_2)_2$ | $C_2H_5$ | Smp. 74-76° |
| 4.30 | $C_2H_5$ | 0 | $(CH_2)_2$ | Cyclopropyl | $n_D^{30}$ 1.5653 |
| 4.31 | $C_2H_5$ | 0 | $(CH_2)_2$ | $C_3H_7$-n | Smp. 60-61° |

Tabelle 4 (Fortsetzung)

| No. | $R_1$ | n | A | $R_2$ | phys. Daten |
|---|---|---|---|---|---|
| 4.32 | $C_2H_5$ | 0 | $(CH_2)_2$ | $C_2H_5$ | Smp. 50-51° |
| 4.33 | $C_2H_5$ | 0 | $(CH_2)_2$ | 2,4-Dichlorphenyl | Smp. 80-82° |
| 4.34 | $CH_3$ | 0 | $(CH_2)_5$ | $C_2H_5$ | Smp. 69-79° |
| 4.35 | $CH_3$ | 0 | $(CH_2)_5$ | 2,4-Dichlorphenyl | Smp. 103-106° |
| 4.36 | $C_2H_5$ | 0 | $(CH_2)_3$ | $C_2H_5$ | $n_D^{30}$ 1.5487 |
| 4.37 | $CH(CH_3)_2$ | 0 | $(CH_2)_2$ | $C_2H_5$ | $n_D^{30}$ 1.5398 |
| 4.38 | $CH(CH_3)_2$ | 0 | $(CH_2)_2$ | $C_3H_7$-n | $n_D^{30}$ 1.5338 |
| 4.39 | $CH(CH_3)_2$ | 0 | $(CH_2)_2$ | Cyclopropyl | $n_D^{30}$ 1.5590 |
| 4.40 | $C_3H_7$-n | 0 | $(CH_2)_2$ | Cyclopropyl | $n_D^{30}$ 1.5574 |
| 4.41 | $C_3H_7$-n | 0 | $(CH_2)_2$ | $CH(CH_3)_2$ | $n_D^{30}$ 1.5339 |
| 4.42 | $C_3H_7$-n | 0 | $(CH_2)_2$ | $C_3H_7$-n | Kp. 153-155°/ 0.013 mbar |
| 4.43 | $C_3H_7$-n | 0 | $(CH_2)_2$ | $C_2H_5$ | Kp. 150-152°/ 0.013 mbar |
| 4.44 | Benzyl | 0 | $(CH_2)_2$ | $C_2H_5$ | $n_D^{30}$ 1.5834 |
| 4.45 | Benzyl | 0 | $(CH_2)_2$ | Cyclopropyl | Smp. 112-113° |
| 4.46 | Benzyl | 0 | $(CH_2)_2$ | $C_3H_7$-n | $n_D^{30}$ 1.5752 |
| 4.47 | $CH_3$ | 0 | $(CH_2)_2$ | $C_4H_9$-n | |
| 4.48 | $CH_3$ | 0 | $(CH_2)_2$ | $CH_3$ | Smp. 80-82° |
| 4.49 | $CH_3$ | 1 | $(CH_2)_2$ | $C_2H_5$ | |
| 4.50 | $CH_3$ | 1 | $(CH_2)_2$ | $C_3H_7$-n | |
| 4.51 | $CH_3$ | 2 | $(CH_2)_2$ | $C_2H_5$ | |
| 4.52 | $CH_3$ | 2 | $(CH_2)_2$ | $C_3H_7$-n | |
| 4.53 | $CH_3$ | 1 | $(CH_2)_3$ | $C_2H_5$ | |
| 4.54 | $CH_3$ | 2 | $(CH_2)_3$ | $C_2H_5$ | |
| 4.55 | $CH_3$ | 1 | $(CH_2)_3$ | $C_3H_7$-n | |
| 4.56 | $CH_3$ | 2 | $(CH_2)_3$ | $C_3H_7$-n | |

Beispiel 6

Herstellung von 5-(1-Methylthiocyclobutan-1-yl)-2-(3-oxa-4-aza-oct-4-en-5-yl)-cyclohexan-1,3-dion [5-(1- Methylthiocyclobutan-1-yl)-2-(1-äthoximino-butyryl)-cyclohexan-1,3-dion]

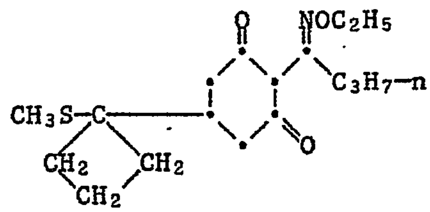

Ein Gemisch von 3,0 g 5-(1-Methylthiocyclobutan-1-yl)-2-n-butyryl-cyclohexan-1,3-dion (Beispiel 4), 1,15 g O-Aethylhydroxylamin-Hydrochlorid und 1,5 g Kaliumcarbonat in 30 ml Chloroform und 3 ml Methanol wird während 3 Tagen bei Raumtemperatur gerührt. Dann dampft man das Reaktionsgemisch zur Trockne ein, nimmt den Rückstand in Ether auf und wäscht die Aetherschicht zuerst mit Wasser, dann mit 1N Salzsäure. Die Aetherschicht wird dann mit 2N Kalilauge kalt extrahiert und die wässrige Schicht mit Aether nachgewaschen. Das basisch-wässrige Extrakt wird kalt gestellt und mit eiskalter halb-conc. Salzsäure bis pH 5,5 neutralisiert. Anschliessend wird wieder mit Aether extrahiert, die Aetherschicht über Natriumsulfat getrocknet, filtriert, eingedampft und der Rückstand in Pentan aufgenommen. Die Pentanllösung wird mit Aktivkohle behandelt, filtriert und eingedampft. Man erhält so 2,5 g Titelprodukt als farbloses Oel. $N_D^{30}$ 1.5428.

In analoger Weise zum Beispiel 6 werden die in der Tabelle 5 aufgeführten Oximäther von 2-Acyl-1,3-cyclohexandionen der Formel Ib hergestellt.

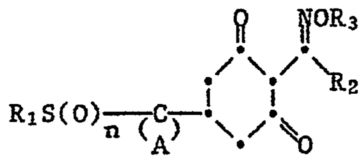

(Ib)

Tabelle 5

| No. | $R_1$ | n | A | $R_2$ | $R_3$ | phys. Daten |
|---|---|---|---|---|---|---|
| 5.01 | $CH_3$ | 0 | $(CH_2)_3$ | $C_3H_7$-n | $C_2H_5$ | $n_D^{30}$ 1.5428 |
| 5.02 | $CH_3$ | 0 | $(CH_2)_5$ | $C_3H_7$-n | $C_2H_5$ | $n_D^{30}$ 1.5429 |
| 5.03 | $CH_3$ | 0 | $(CH_2)_3$ | $C_3H_7$-n | $CH_2CH{=}CH_2$ | $n_D^{30}$ 1.5468 |
| 5.04 | $CH_3$ | 0 | $(CH_2)_3$ | $C_3H_7$-n | $CH_2CH{=}CHCl$ | $n_D^{31}$ 1.5541 |
| 5.05 | $C_2H_5$ | 0 | $(CH_2)_3$ | $C_3H_7$-n | $C_2H_5$ | $n_D^{30}$ 1.5356 |
| 5.06 | $C_2H_5$ | 0 | $(CH_2)_3$ | $C_2H_5$ | $C_2H_5$ | $n_D^{30}$ 1.5410 |
| 5.07 | $C_2H_5$ | 0 | $(CH_2)_3$ | $C_3H_7$-n | $C_4H_9$-n | |
| 5.08 | $C_2H_5$ | 2 | $(CH_2)_3$ | $C_3H_7$-n | $C_2H_5$ | |
| 5.09 | $CH_3$ | 2 | $(CH_2)_3$ | $C_3H_7$-n | $C_2H_5$ | |
| 5.10 | $C_2H_5$ | 0 | $(CH_2)_2$ | $C_3H_7$-n | $CH_2CH{=}CH_2$ | $n_D^{30}$ 1.5360 |
| 5.11 | $C_3H_7$-n | 0 | $(CH_2)_2$ | $C_2H_5$ | $C_2H_5$ | $n_D^{30}$ 1.5312 |
| 5.12 | $C_2H_5$ | 0 | $(CH_2)_2$ | $C_3H_7$-n | $CH_2CH{=}CHCl$ | $n_D^{30}$ 1.5460 |
| 5.13 | $CH_3$ | 0 | $(CH_2)_4$ | $C_3H_7$-n | $C_2H_5$ | $n_D^{30}$ 1.5426 |
| 5.14 | $C_2H_5$ | 0 | $(CH_2)_4$ | $C_3H_7$-n | $C_2H_5$ | |
| 5.15 | $C_2H_5$ | 0 | $(CH_2)_4$ | $C_2H_5$ | $C_2H_5$ | |
| 5.16 | $C_2H_5$ | 0 | $(CH_2)_4$ | $C_3H_7$-n | $CH_3$ | |
| 5.17 | $C_2H_5$ | 2 | $(CH_2)_4$ | $C_2H_5$ | $C_4H_9$-n | |
| 5.18 | $C_2H_5$ | 1 | $(CH_2)_4$ | $C_3H_7$-n | $C_2H_5$ | |
| 5.19 | $C_2H_5$ | 0 | $(CH_2)_5$ | $C_3H_7$-n | $C_2H_5$ | $n_D^{30}$ 1.5398 |
| 5.20 | $C_2H_5$ | 0 | $(CH_2)_5$ | $C_2H_5$ | $C_2H_5$ | $n_D^{30}$ 1.5450 |
| 5.21 | $C_2H_5$ | 0 | $(CH_2)_5$ | $C_3H_7$-n | $CH_2CH{=}CH_2$ | $n_D^{30}$ 1.5449 |
| 5.22 | $CH_3$ | 0 | $(CH_2)_5$ | $C_3H_7$-n | $CH_2CH{=}CHCl$ | $n_D^{30}$ 1.5571 |
| 5.23 | $CH_3$ | 0 | $(CH_2)_4$ | $C_3H_7$-n | $CH_2CH{=}CHCl$ | $n_D^{30}$ 1.5569 |

Tabelle 5 (Fortsetzung)

| No. | $R_1$ | n | A | $R_2$ | $R_3$ | phys. Daten |
|---|---|---|---|---|---|---|
| 5.24 | $CH_3$ | 0 | $(CH_2)_4$ | $C_3H_7$-n | $CH_2CH=CH_2$ | $n_D^{30}$ 1.5480 |
| 5.25 | $CH_3$ | 0 | $(CH_2)_3$ | $C_3H_7$-n | $C_4H_9$-n | $n_D^{30}$ 1.5324 |
| 5.26 | $CH_3$ | 0 | $(CH_2)_3$ | $C_2H_5$ | $C_4H_9$-n | $n_D^{30}$ 1.5383 |
| 5.27 | $CH_3$ | 0 | $(CH_2)_2$ | $C_3H_7$-n | $C_2H_5$ | $n_D^{30}$ 1.5362 |
| 5.28 | $CH_3$ | 0 | $(CH_2)_2$ | $C_3H_7$-n | $CH_2CH=CH_2$ | $n_D^{30}$ 1.5420 |
| 5.29 | $CH_3$ | 0 | $(CH_2)_2$ | $C_3H_7$-n | $CH_2CH=CHCl$ | $n_D^{30}$ 1.5514 |
| 5.30 | $C_2H_5$ | 0 | $(CH_2)_2$ | $C_3H_7$-n | $C_2H_5$ | $n_D^{30}$ 1.5302 |
| 5.31 | $CH_3$ | 0 | $(CH_2)_2$ | $C_2H_5$ | $C_2H_5$ | Smp. 65-69° |
| 5.32 | $C_2H_5$ | 0 | $(CH_2)_2$ | $C_2H_5$ | $C_2H_5$ | $n_D^{30}$ 1.5359 |
| 5.33 | $C_2H_5$ | 0 | $(CH_2)_2$ | $C_2H_5$ | $C_4H_9$-n | $n_D^{30}$ 1.5288 |
| 5.34 | $C_2H_5$ | 0 | $(CH_2)_3$ | $C_2H_5$ | $CH_2CH=CHCl$ | $n_D^{30}$ 1.5558 |
| 5.35 | $C_2H_5$ | 0 | $(CH_2)_3$ | $C_3H_7$-n | $CH_2CH=CHCl$ | $n_D^{30}$ 1.5496 |
| 5.36 | $CH_3$ | 0 | $(CH_2)_3$ | $C_2H_5$ | $CH_3$ | $n_D^{30}$ 1.5539 |
| 5.37 | $CH_3$ | 0 | $(CH_2)_3$ | $C_2H_5$ | $C_2H_5$ | $n_D^{30}$ 1.5469 |
| 5.38 | $CH_3$ | 0 | $(CH_2)_3$ | $C_2H_5$ | $CH_2CH=CHCl$ | $n_D^{30}$ 1.5613 |
| 5.39 | $CH_3$ | 0 | $(CH_2)_2$ | $C_2H_5$ | $CH_2CH=CHCl$ | $n_D^{30}$ 1.5568 |
| 5.40 | $C_2H_5$ | 0 | $(CH_2)_2$ | $C_3H_7$-n | $C_4H_9$-n | $n_D^{30}$ 1.5242 |
| 5.41 | $C_2H_5$ | 0 | $(CH_3)_2$ | $C_2H_5$ | $CH_2CH=CH_2$ | $n_D^{30}$ 1.5407 |
| 5.42 | $C_2H_5$ | 0 | $(CH_3)_2$ | $C_2H_5$ | $CH_2CH=CHCl$ | $n_D^{30}$ 1.5514 |
| 5.43 | $CH_3$ | 0 | $(CH_2)_5$ | $C_3H_7$-n | $C_4H_9$-n | $n_D^{30}$ 1.5379 |
| 5.44 | $CH_3$ | 0 | $(CH_2)_5$ | $C_2H_5$ | $C_2H_5$ | $n_D^{30}$ 1.5496 |
| 5.45 | $CH_3$ | 0 | $(CH_2)_5$ | $C_2H_5$ | $CH_2CH=CH_2$ | $n_D^{30}$ 1.5547 |
| 5.46 | $CH_3$ | 0 | $(CH_2)_5$ | $C_2H_5$ | $CH_2CH=CHCl$ | $n_D^{30}$ 1.5631 |
| 5.47 | $CH_3$ | 0 | $(CH_2)_5$ | $C_2H_5$ | $C_4H_9$-n | $n_D^{30}$ 1.5415 |
| 5.48 | $CH_3$ | 0 | $(CH_2)_5$ | $C_2H_5$ | $CH_3$ | $n_D^{30}$ 1.5565 |

Tabelle 5 (Fortsetzung)

| No. | $R_1$ | n | A | $R_2$ | $R_3$ | phys. Daten |
|---|---|---|---|---|---|---|
| 5.49 | $C_2H_5$ | 0 | $(CH_2)_5$ | $C_3H_7$-n | $CH_2CH$=CHCl | $n_D^{30}$ 1.5526 |
| 5.50 | $CH_3$ | 0 | $(CH_2)_2$ | $C_2H_5$ | $CH_2CH$=CHCl | (trans) $n_D^{30}$ 1.5578 |
| 5.51 | $CH_3$ | 0 | $(CH_3)_2$ | $C_2H_5$ | $CH_2CH$=CHCl | (cis) $n_D^{30}$ 1.5570 |
| 5.52 | $C_2H_5$ | 0 | $(CH_2)_2$ | $C_2H_5$ | $CH_2CH$=CHCl | (trans) $n_D^{30}$ 1.5519 |
| 5.53 | $C_2H_5$ | 0 | $(CH_2)_2$ | $C_3H_7$-n | $CH_2CH$=CHCl | (trans) $n_D^{30}$ 1.5464 |
| 5.54 | $C_2H_5$ | 0 | $(CH_2)_2$ | $C_2H_5$ | $CH_2CH$=CHCl | (cis) $n_D^{30}$ 1.5511 |
| 5.55 | $C_2H_5$ | 0 | $(CH_2)_2$ | $C_3H_7$-n | $CH_2CH$=CHCl | (cis) $n_D^{30}$ 1.5460 |
| 5.56 | $CH_3$ | 0 | $(CH_2)_2$ | $C_2H_5$ | $CH_3$ | $n_D^{30}$ 1.5491 |
| 5.57 | $CH_3$ | 0 | $(CH_2)_2$ | $C_2H_5$ | $CH_2CH$=$CH_2$ | Smp. 46-49° |
| 5.58 | $CH_3$ | 0 | $(CH_2)_2$ | $C_3H_7$-n | $CH_2CH$=CHCl | (trans) $n_D^{30}$ 1.5516 |
| 5.59 | $CH_3$ | 0 | $(CH_2)_2$ | $C_3H_7$-n | $CH_2CH$=CHCl | (cis) $n_D^{30}$ 1.5510 |
| 5.60 | $CH(CH_3)_2$ | 0 | $(CH_2)_2$ | $C_2H_5$ | $C_2H_5$ | $n_D^{30}$ 1.5315 |
| 5.61 | $CH(CH_3)_2$ | 0 | $(CH_2)_2$ | $C_2H_5$ | $CH_2CH$=$CH_2$ | $n_D^{30}$ 1.5372 |
| 5.62 | $CH(CH_3)_2$ | 0 | $(CH_2)_2$ | $C_2H_5$ | $CH_2CH$=CHCl | (trans) $n_D^{30}$ 1.5469 |
| 5.63 | $CH(CH_3)_2$ | 0 | $(CH_2)_2$ | $C_3H_7$-n | $C_2H_5$ | $n_D^{30}$ 1.5258 |
| 5.64 | $CH(CH_3)_2$ | 0 | $(CH_2)_2$ | $C_3H_7$-n | $CH_2CH$=$CH_2$ | $n_D^{30}$ 1.5322 |
| 5.65 | $CH(CH_3)_2$ | 0 | $(CH_2)_2$ | $C_3H_7$-n | $CH_2CH$=CHCl | (trans) $n_D^{30}$ 1.5431 |
| 5.66 | $CH(CH_3)_2$ | 0 | $(CH_2)_2$ | $C_3H_7$-n | $CH_3$ | $n_D^{30}$ 1.5313 |
| 5.67 | $CH_3$ | 0 | $(CH_2)_2$ | $C_2H_5$ | $CH_2CCl$=$CH_2$ | $n_D^{30}$ 1.5538 |

Tabelle 5 (Fortsetzung)

| No. | $R_1$ | n | A | $R_2$ | $R_3$ | phys. Daten |
|---|---|---|---|---|---|---|
| 5.68 | $C_3H_7$-n | 0 | $(CH_2)_2$ | $C_2H_5$ | $CH_2CH{=}CHCl$ | (trans) $n_D^{30}$ 1.5472 |
| 5.69 | $C_3H_7$-n | 0 | $(CH_2)_2$ | $C_2H_5$ | $CH_2CCl{=}CH_2$ | $n_D^{30}$ 1.5440 |
| 5.70 | $C_3H_7$-n | 0 | $(CH_2)_2$ | $C_2H_5$ | $CH_2CH{=}CH_2$ | $n_D^{30}$ 1.5370 |
| 5.71 | $C_3H_7$-n | 0 | $(CH_2)_2$ | $C_3H_7$-n | $C_2H_5$ | $n_D^{30}$ 1.5268 |
| 5.72 | $C_3H_7$-n | 0 | $(CH_2)_2$ | $C_3H_7$-n | $CH_2CH{=}CHCl$ | (trans) $n_D^{30}$ 1.5420 |
| 5.73 | $C_3H_7$-n | 0 | $(CH_2)_2$ | $C_3H_7$-n | $CH_2CCl{=}CH_2$ | $n_D^{30}$ 1.5390 |
| 5.74 | $C_3H_7$-n | 0 | $(CH_2)_2$ | $C_3H_7$-n | $C_4H_9$-n | $n_D^{30}$ 1.5208 |
| 5.75 | $CH_3$ | 0 | $(CH_2)_2$ | $C_2H_5$ | $CH_2CBr{=}CH_2$ | |
| 5.76 | Benzyl | 0 | $(CH_2)_2$ | $C_2H_5$ | $C_2H_5$ | Smp. 115-117° |
| 5.77 | Benzyl | 0 | $(CH_2)_2$ | $C_2H_5$ | $CH_2CH{=}CHCl$ | (trans) $n_D^{30}$ 1.5792 |
| 5.78 | Benzyl | 0 | $(CH_2)_2$ | $C_3H_7$-n | $C_2H_5$ | $n_D^{30}$ 1.5478 |
| 5.79 | Benzyl | 0 | $(CH_2)_2$ | $C_3H_7$-n | $CH_2CH{=}CHCl$ | (trans) $n_D^{30}$ 1.5706 |
| 5.80 | $CH_3$ | 0 | $(CH_2)_2$ | $C_2H_5$ | $C_3H_7$-n | |
| 5.81 | $CH_3$ | 0 | $(CH_2)_2$ | $C_2H_5$ | $C_4H_9$-n | $n_D^{30}$ 1.5323 |
| 5.82 | $CH_3$ | 0 | $(CH_2)_2$ | $C_2H_5$ | $CH_2C{\equiv}CH$ | $n_D^{30}$ 1.5533 |
| 5.83 | $CH_3$ | 1 | $(CH_2)_2$ | $C_2H_5$ | $CH_2CH{=}CHCl$ | (trans) |
| 5.84 | $CH_3$ | 2 | $(CH_2)_2$ | $C_2H_5$ | $CH_2CH{=}CHCl$ | (trans) |
| 5.85 | $CH_3$ | 0 | $(CH_2)_2$ | $C_3H_7$-n | $CH_2CCl{=}CH_2$ | $n_D^{30}$ 1.5478 |
| 5.86 | $CH_3$ | 0 | $(CH_2)_2$ | $C_3H_7$-n | $C_3H_7$-n | |
| 5.87 | $CH_3$ | 0 | $(CH_2)_2$ | $C_3H_7$-n | $CH_2C{\equiv}CH$ | $n_D^{30}$ 1.5476 |
| 5.88 | $CH_3$ | 0 | $(CH_2)_2$ | $CH_3$ | $CH_3$ | |
| 5.89 | $CH_3$ | 0 | $(CH_2)_2$ | $C_2H_5$ | $CH_2CH{=}CHBr$ | (trans) |
| 5.90 | $CH_3$ | 0 | $(CH_2)_2$ | $C_2H_5$ | $CH_2CH{=}CHBr$ | $n_D^{30}$ 1.5679 |
| 5.91 | $CH_3$ | 0 | $(CH_2)_2$ | $CH_3$ | $C_2H_5$ | $n_D^{30}$ 1.5469 |
| 5.92 | $CH_3$ | 0 | $(CH_2)_2$ | $C_3H_7$-n | $CH_2CH{=}CHBr$ | (trans) |
| 5.93 | $CH_3$ | 0 | $(CH_2)_2$ | $C_3H_7$-n | $CH_2CH{=}CHBr$ | $n_D^{30}$ 1.5619 |

Tabelle 5 (Fortsetzung)

| No. | $R_1$ | n | A | $R_2$ | $R_3$ | phys. Daten |
|---|---|---|---|---|---|---|
| 5.94 | $CH_3$ | 0 | $(CH_2)_2$ | $CH_3$ | $CH_2CH=CHCl$ | (trans) $n_D^{30}$ 1.5637 |
| 5.95 | $CH_3$ | 0 | $(CH_2)_3$ | $C_2H_5$ | $CH_2CH=CHCl$ | (trans) |
| 5.96 | $CH_3$ | 0 | $(CH_2)_3$ | $C_3H_7$-n | $CH_2CH=CHCl$ | (trans) |
| 5.97 | $CH_3$ | 1 | $(CH_2)_2$ | $C_3H_7$-n | $C_2H_5$ | |
| 5.98 | $CH_3$ | 2 | $(CH_2)_2$ | $C_3H_7$-n | $C_2H_5$ | |
| 5.99 | $CH_3$ | 0 | $(CH_2)_2$ | $C_4H_9$-n | $CH_2CH=CHCl$ | (trans) |
| 5.100 | $CH_3$ | 0 | $(CH_2)_2$ | $C_4H_9$-n | $C_2H_5$ | |
| 5.101 | $CH_3$ | 0 | $(CH_2)_2$ | $C_2H_5$ | $CH_2CH=CCl_2$ | |
| 5.102 | $C_2H_5$ | 0 | $(CH_2)_2$ | $C_2H_5$ | $CH_2CH=CCl_2$ | |
| 5.103 | $CH_3$ | 0 | $(CH_2)_2$ | $C_3H_7$-n | $CH_2CH=CCl_2$ | |
| 5.104 | $C_2H_5$ | 0 | $(CH_2)_2$ | $C_3H_7$-n | $CH_2CH=CCl_2$ | |
| 5.105 | $CH_3$ | 0 | $(CH_2)_2$ | $C_2H_5$ | $CH_2CCl=CHCl$ | |
| 5.106 | $C_2H_5$ | 0 | $(CH_2)_2$ | $C_2H_5$ | $CH_2CCl=CHCl$ | |
| 5.107 | $CH_3$ | 0 | $(CH_2)_2$ | $C_3H_7$-n | $CH_2CCl=CHCl$ | |
| 5.108 | $C_2H_5$ | 0 | $(CH_2)_2$ | $C_3H_7$-n | $CH_2CCl=CHCl$ | |
| 5.109 | $CH_3$ | 0 | $(CH_2)_2$ | $C_2H_5$ | $CH_2CH=CHCH_3$ | (trans) $n_D^{30}$ 1.5450 |
| 5.110 | $C_2H_5$ | 0 | $(CH_2)_2$ | $C_2H_5$ | $CH_2CH=CHCH_3$ | (trans) |
| 5.111 | $CH_3$ | 0 | $(CH_2)_2$ | $C_3H_7$-n | $CH_2CH=CHCH_3$ | (trans) $n_D^{30}$ 1.5401 |
| 5.112 | $C_2H_5$ | 0 | $(CH_2)_2$ | $C_3H_7$-n | $CH_2CH=CHCH_3$ | (trans) |
| 5.113 | $CH_3$ | 0 | $(CH_3)_2$ | $C_2H_5$ | $CH_2CH=CClCH_3$ | (trans) |
| 5.114 | $CH_3$ | 0 | $(CH_3)_2$ | $C_2H_5$ | $CH_2CH=CClCH_3$ | (cis) |
| 5.115 | $C_2H_5$ | 0 | $(CH_3)_2$ | $C_2H_5$ | $CH_2CH=CClCH_3$ | (trans) |
| 5.116 | $C_2H_5$ | 0 | $(CH_3)_2$ | $C_2H_5$ | $CH_2CH=CClCH_3$ | (cis) |
| 5.117 | $CH_3$ | 0 | $(CH_2)_2$ | $C_3H_7$-n | $CH_2CH=CClCH_3$ | (trans) |
| 5.118 | $CH_3$ | 0 | $(CH_2)_2$ | $C_3H_7$-n | $CH_2CH=CClCH_3$ | (cis) |
| 5.119 | $C_2H_5$ | 0 | $(CH_2)_2$ | $C_3H_7$-n | $CH_2CH=CClCH_3$ | (trans) |
| 5.120 | $C_2H_5$ | 0 | $(CH_2)_2$ | $C_3H_7$-n | $CH_2CH=CClCH_3$ | (cis) |
| 5.121 | $CH_3$ | 0 | $(CH_2)_2$ | $C_2H_5$ | $CH_2CHClCH=CH_2$ | |
| 5.122 | $C_2H_5$ | 0 | $(CH_2)_2$ | $C_2H_5$ | $CH_2CHClCH=CH_2$ | |
| 5.123 | $CH_3$ | 0 | $(CH_2)_2$ | $C_3H_7$-n | $CH_2CHClCH=CH_2$ | |
| 5.124 | $C_2H_5$ | 0 | $(CH_2)_2$ | $C_3H_7$-n | $CH_2CHClCH=CH_2$ | |
| 5.125 | $CH_3$ | 0 | $(CH_2)_2$ | $C_2H_5$ | $CH_2CH=CHCH_2Cl$ | |
| 5.126 | $CH_3$ | 0 | $(CH_2)_2$ | $C_3H_7$-n | $CH_2CH=CHCH_2Cl$ | |

Tabelle 5 (Fortsetzung)

| No. | $R_1$ | n | A | $R_2$ | $R_3$ | phys. Daten |
|---|---|---|---|---|---|---|
| 5.127 | $CH_3$ | 0 | $(CH_2)_2$ | $C_2H_5$ | $CH_2CH_2CH_2Cl$ | |
| 5.128 | $CH_3$ | 0 | $(CH_2)_2$ | $C_3H_7$-n | $CH_2CH_2CH_2Cl$ | |
| 5.129 | $CH_3$ | 0 | $(CH_2)_2$ | $C_2H_5$ | $CH_2CH_2Cl$ | |
| 5.130 | $CH_3$ | 0 | $(CH_2)_2$ | $C_2H_5$ | $CH_2CH(CH_3)_2$ | |
| 5.131 | $CH_3$ | 0 | $(CH_2)_2$ | $C_3H_7$-n | $CH_2CH(CH_3)_2$ | |
| 5.132 | $CH_3$ | 0 | $(CH_2)_2$ | $CH_2OCH_3$ | $CH_2CH{=}CHCl$ | (trans) |
| 5.133 | $CH_3$ | 0 | $(CH_2)_2$ | Benzyl | $CH_2CH{=}CHCl$ | (trans) |
| 5.134 | $CH_3$ | 0 | $(CH_2)_2$ | Phenyl-ethyl | $CH_2CH{=}CHCl$ | (trans) |
| 5.135 | $CH_3$ | 0 | $(CH_2)_2$ | $C_2F_5$ | $C_2H_5$ | |
| 5.136 | $CH_3$ | 0 | $(CH_2)_2$ | $C_2F_5$ | $CH_2CH{=}CHCl$ | (trans) |
| 5.137 | $CH_3$ | 0 | $(CH_2)_2$ | $C_3F_7$-n | $CH_2CH{=}CHCl$ | (trans) |
| 5.138 | $CH_3$ | 0 | $(CH_2)_2$ | $C_3F_7$-n | $C_2H_5$ | |
| 5.139 | $CH_3$ | 0 | $(CH_2)_2$ | $CF_3$ | $C_2H_5$ | |
| 5.140 | $CH_3$ | 0 | $(CH_2)_2$ | $C_2H_5$ | $CH_2CCl{=}CClCH_3$ | (trans) |
| 5.141 | $CH_3$ | 0 | $(CH_2)_2$ | $C_3H_7$-n | $CH_2CCl{=}CClCH_3$ | (trans) |
| 5.142 | $CH_3$ | 0 | $(CH_2)_2$ | $C_2H_5$ | $CH_2CCl{=}CClCH_3$ | (cis) |
| 5.143 | $CH_3$ | 0 | $(CH_2)_2$ | $C_3H_7$-n | $CH_2CCl{=}CClCH_3$ | (cis) |
| 5.144 | $CH_3$ | 0 | $(CH_2)_5$ | $C_3H_7$-n | $CH_2$-$CH{=}CH_2$ | $n_D^{30}$ 1.5491 |
| 5.145 | $CH_3$ | 0 | $(CH_2)_2$ | $CH_3$ | $CH_2$-$CCl{=}CH_2$ | $n_D^{30}$ 1.5592 |
| 5.146 | Benzyl | 0 | $(CH_2)_2$ | $C_2H_5$ | $C_4H_9$-n | Smp. 57-59° |
| 5.147 | Benzyl | 0 | $(CH_2)_2$ | $C_2H_5$ | $CH_2CH{=}CH_2$ | Smp. 85-87° |
| 5.148 | Benzyl | 0 | $(CH_2)_2$ | $C_3H_7$-n | $C_4H_9$-n | $n_D^{30}$ 1.5472 |
| 5.149 | Benzyl | 0 | $(CH_2)_2$ | $C_3H_7$-n | $CH_2$-$CH{=}CH_2$ | $n_D^{30}$ 1.5490 |
| 5.150 | $CH_3$ | 0 | $(CH_2)_2$ | $C_2H_5$ | $CH_2$-$C{\equiv}C$-$CH_3$ | |
| 5.151 | $CH_3$ | 0 | $(CH_2)_3$ | $C_2H_5$ | $CH_2$-$CH{=}CHBr$ | |
| 5.152 | $CH_3$ | 0 | $(CH_2)_3$ | $C_2H_5$ | $CH_2$-$C{\equiv}CH$ | |
| 5.153 | $CH_3$ | 0 | $(CH_2)_3$ | $C_2H_5$ | $CH_2CCl{=}CH_2$ | |
| 5.154 | $CH_3$ | 0 | $(CH_2)_3$ | $C_2H_5$ | $CH_2$-$CH{=}CHCH_3$ | (trans) |
| 5.155 | $CH_3$ | 0 | $(CH_2)_3$ | $C_3H_7$(n) | $CH_2$-$CH{=}CHBr$ | |
| 5.156 | $CH_3$ | 0 | $(CH_2)_3$ | $C_3H_7$(n) | $CH_2$-$C{\equiv}CH$ | |
| 5.157 | $CH_3$ | 0 | $(CH_2)_3$ | $C_3H_7$(n) | $CH_2$-$CCl{=}CH_2$ | |
| 5.158 | $CH_3$ | 0 | $(CH_2)_3$ | $C_3H_7$(n) | $CH_2$-$CH{=}CH$-$CH_3$ | (trans) |

Beispiel 7: Formulierungsbeispiele für Wirkstoffe der Formel I (% = Gewichtsprozent)

| a) Spritzpulver | a) | b) | c) |
|---|---|---|---|
| Wirkstoff der Tabelle 4 oder 5 | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthylenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff der Tabelle 4 oder 5 | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff der Tabelle 4 oder 5 | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder-Granulat | a) | b) |
|---|---|---|
| Wirkstoff der Tabelle 4 oder 5 | 10% | 1 % |
| Na-Ligninsulfonat | 2% | 2 % |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungsgranulat | |
|---|---|
| Wirkstoff der Tabelle 4 oder 5 | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff der Tabelle 4 oder 5 | 40% | 5% |
| Aethylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff der Tabelle 4 oder 5 | 5% |
| Isopropylamin | 1% |
| Octylphenolpolyäthylenglykoläther (78 Mol AeO) | 3% |
| Wasser | 91% |

<u>Beispiel 8: Herbizidwirkung vor dem Auflaufen der Pflanzen</u>

Im Gewächshaus werden Pflanzensamen in Blumentöpfe von 11 cm Durchmesser gesät. Unmittelbar danach wird die Erdoberfläche mit einer wässrigen Emulsion der Wirkstoffe behandelt. Es wird eine Konzentration von 4 kg Wirkstoffmenge pro Hektar angewendet. Die Töpfe werden dann im Gewächshaus bei einer Temperatur von 22-25°C und 50-70 % relativer Luftfeuchtigkeit gehalten. Nach 3 Wochen wird der Versuch ausgewertet und die Wirkung auf die Versuchspflanzen bewertet. Die geprüften Verbindungen der Tabellen 4 und 5 zeigen dabei gute Wirkung vornehmlich gegen die monokotylen Testpflanzen.

<u>Beispiel 9: Herbizide Wirkung bei Applikation der Wirkstoffe nach dem Auflaufen der Pflanzen</u>

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässrigen Wirkstoffemulsionen (erhalten aus dem 25%igen Emulsionskonzentrat) in einer Dosierung von 4 kg/ha gespritzt. Die behandelten Pflanzen werden dann bei optimalen Bedingungen von Licht, regelmässigem Begiessen, 22-25°C Temperatur und 50-70 %iger relativer Luftfeuchtigkeit gehalten. Die Auswertung des Versuches erfolgt 15 Tage nach der Behandlung. Die geprüften Verbindungen zeigen in diesem Versuch gute Wirkung.

<u>Beispiel 10: Wuchshemmung bei tropischen Bodenbedecker-Leguminosen (cover crops).</u>

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21 °C bei Nach gehalten. 4 Wochen nach der Applikation wird der Veruch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 4 und 5 behandelten Pflanzen eine deutliche Reduktion des Neuzuwachses (wengier als 20 % des Neuzuwachses bei unbehandelten Kontrollpflanzen), ohne dass dabei die Versuchspflanzen geschädigt werden.

Beispiel 11: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 2000 g AS/ha. Die Auswertung erfolgt ca. 5 Wochen nach der Applikation des Wirkstoffs. Im Vergleich zu unbehandelten Kontrollpflanzen zeigen die mit erfindungsgemässen Wirkstoffe der Tabellen 4 und 5 behandelten Pflanzen eine merkliche Erhöhung der Anzahl und des Gewichts der Schoten.

Beispiel 12: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 3000 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 13: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem Letzten Schnitt mit der wässrigen Spritzbrühe eines Wirkstoffes der Formel I besprüht. Die Wirkstoffmenge beträgt umgerechnet bis zu 3000 g Aktivsubstanz pro Hektar. 21 Tage nach Applikation wird das Wachstum der Gräser beurteilt. Die geprüften Verbindungen der Tabellen 4 und 5 bewirken eine Reduktion des Neuzuwachses von um 10-30 % im Vergleich zur unbehandelten Kontrolle.

## Patentansprüche

1. Neue 2-Acyl-1,3-cyclohexandione und deren Oximäther der Formel I

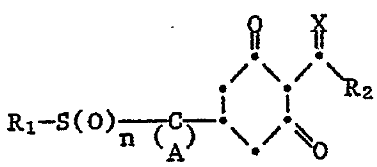

(I).

worin A eine 2-7-gliedrige Alkylenbrücke, eine 3-7-gliedrige Alkenylenbrücke, welche ein- oder mehrfach ungesättigt sein kann,
n Null eins oder zwei
$R_1$ $C_1$-$C_4$-Alkyl oder Benzyl
$R_2$ $C_1$-$C_6$-Alkyl, unsubstituiert oder substituiert durch Halogen, $C_1$-$C_4$-Alkoxy, $C_1$-$C_4$-Alkylthio; $C_3$-$C_6$-Cycloalkyl; Phenyl, Benzyl oder Phenyläthyl, wobei der Phenylring durch Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann,
X Sauerstoff oder ein Rest -$NOR_3$ und
$R_3$ $C_1$-$C_6$-Alkyl, $C_1$-$C_6$-Halogenalkyl, $C_3$-$C_6$-Alkenyl, $C_3$-$C_6$-Halogenalkenyl oder $C_3$-$C_6$-Alkinyl
bedeuten, sowie die Salze dieser Verbindungen mit Metallen und Stickstoffbasen.

2. Acylcyclohexandione gemäss Anspruch 1 der Formel Ia,

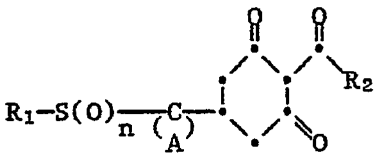

(Ia)

worin
A eine 2-7 gliedrige Alkylenbrücke,
n Null, eins oder zwei,
$R_1$ $C_1$-$C_4$-Alkyl oder Benzyl und
$R_2$ $C_1$-$C_6$-Alkyl, unsubstituiert oder substituiert durch Halogen, $C_1$-$C_4$-Alkoxy oder $C_1$-$C_4$-Alkylthio; $C_3$-$C_6$-Cycloalkyl; Phenyl, Benzyl oder Phenyläthyl, wobei der Phenylring durch Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Cyan oder Nitro substituiert sein kann
bedeuten.

3. Acylcyclohexandione gemäss Anspruch 2 der Formel Ia worin A die Aethylenbrücke, n Null, $R_1$ Methyl oder Aethyl und $R_2$ Aethyl oder n-Propyl.

4. 5-(1-Methylthiocyclobutan-1-yl)-2-(2,4-dichlorobenzoyl)-cyclohexan-1,3-dion gemäss Anspruch 2.

5. 5-(1-Methylthiocyclobutan-1-yl)-2-n-butyryl-cyclohexan-1,3-dion gemäss Anspruch 2.

6. 5-(1-Methylthiocyclobutan-1-yl)-2-cyclopropylcarbonyl-cyclohexan-1,3-dion gemäss Anspruch 2.

7. 5-(1-Methylthiocyclobutan-1-yl)-2-(2,3-dichlorobenzoyl)-cyclohexan-1,3-dion gemäss Anspruch 2.

8. 5-(1-Methylsulfonylcyclobutan-1-yl)-2-n-butyryl-cyclohexan-1,3-dion gemäss Anspruch 2.

9. 5-(1-Methylthiocyclopropan-1-yl)-2-n-butyryl-cyclohexan-1,3-dion gemäss Anspruch 2.

10. 5-(1-Methylthiocyclopropan-1-yl)-2-propionyl-cyclohexan-1,3-dion gemäss Anspruch 2.

11. 5-(1-Aethylthiocyclopropan-1-yl)-2-propionyl-cyclohexan-1,3-dion gemäss Anspruch 2.

12. Acyl-cyclohexandion-Oximäther gemäss Anspruch 1 der Formel Ib

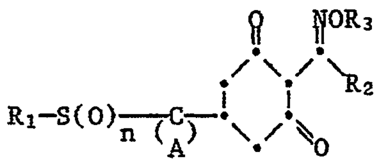

(Ib)

worin
A eine 2-7 gliedrige Alkylenbrücke,
n Null, eins oder zwei,
$R_1$ $C_1$-$C_4$-Alkyl oder Benzyl,
$R_2$ $C_1$-$C_6$-Alkyl, unsubstituiert oder substituiert durch Halogen, $C_1$-$C_4$-Alkoxy oder $C_1$-$C_4$-Alkylthio,
$R_3$ $C_1$-$C_6$-Alkyl, $C_3$-$C_6$-Halogenalkyl, $C_3$-$C_6$-Alkenyl, $C_3$-$C_6$-Halogenalkenyl oder $C_3$-$C_6$-Alkinyl,
bedeuten.

13. Acylcyclohexan-Oximäther gemäss Anspruch 12 der Formel Ib worin A die Aethylenbrücke, n Null, $R_1$ Methyl oder Aethyl, $R_2$ Aethyl oder n-Propyl und $R_3$ cis- oder trans-3-Chlorallyl bedeuten.

14. 5-(1-Methylthiocyclopentan-1-yl)-2-(1-äthoximino-n-butyryl)-cyclohexan-1,3-dion gemäss Anspruch 12.

15. 5-(1-Methylthiocyclobutan-1-yl)-2-(1-äthoximino-n-butyryl)-cyclohexan-1,3-dion gemäss Anspruch 12.

16. 5-(1-Methylthiocyclohexan-1-yl)-2-(1-äthoximino-n-butyryl)-cyclohexan-1,3-dion gemäss Anspruch 12.

17. 5-(1-Methylthiocyclobutan-1-yl)-2-(1-allyloxyimino-n-butyryl)-cyclohexan-1,3-dion gemäss Anspruch 12.

18. 5-(1-Methylthiocyclopropan-1-yl)-2-(1-äthoximino-n-butyryl)-cyclohexan-1,3-dion gemäss Anspruch 12.

19. 5-(1-Methylthiocyclopropan-1-yl)-2-[1-(trans-3-chlorallyloximino)-n-butyryl]-cyclohexan-1,3-dion gemäss Anspruch 12.

20. 5-(1-Methylthiocyclopropan-1-yl)-2-[1-(trans-3-chlorallyloximino)-propionyl]-cyclohexan-1,3-dion gemäss Anspruch 12.

21. 5-(1-Methylthiocyclopropan-1-yl)-2-[1-(cis-3-chlorallyloximino)-propionyl]-cyclohexan-1,3-dion gemäss Anspruch 12.

22. 5-(1-Aethylthiocyclopropan-1-yl)-2-[1-(trans-3-chlorallyloximino)-propionyl]-cyclohexan-1,3-dion gemäss Anspruch 12.

23. 5-(1-Aethylthiocyclopropan-1-yl)-2-[1-(trans-3-chlorallyloximino)-n-butyryl]-cyclohexan-1,3-dion gemäss Anspruch 12.

24. Verfahren zur Herstellung der Acyl-Cyclohexandione und deren Oximäther der Formel I gemäss Anspruch 1, dadurch gekennzeichnet, dass man in einem inerten organischen Lösungsmittel, in Gegenwart der äquimolaren Menge einer Base, ein 1,3-Cyclohexandionderivat der Formel II

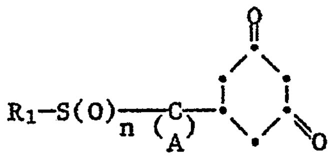

(II)

worin A, n und $R_1$ die im Anspruch 1 gegebene Bedeutung haben, mit einem Säurehalogenid oder Säureanhydrid der Formel III

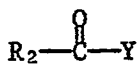

(III)

worin Y ein Halogenatom oder einen Rest

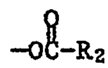

bedeutet und $R_2$ die im Anspruch 1 gegebene Bedeutung hat, den erhaltenen Cyclohexanon-Ester der Formel IV

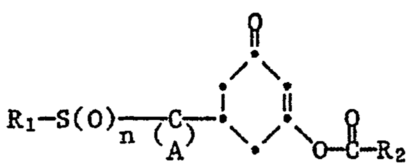

(IV)

worin A, n, $R_1$ und $R_2$ die im Anspruch 1 gegebene Bedeutung haben, in einem inerten organischen Lösungsmittel in Gegenwart eines Katalysators zum 2-Acyl-1,3-cyclohexan-dion-derivat der Formel Ia umlagert,

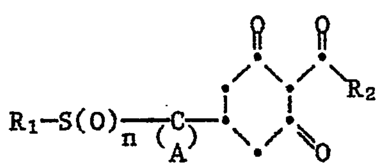

(Ia)

worin A, n, $R_1$ und $R_2$ die im Anspruch 1 gegebene Bedeutung haben, und dieses gewünschtenfalls in einem inerten organischen Lösungsmittel, in Gegenwart der äquimolaren Menge einer Base, mit einem Hydroxylamin • Hydrochlorid der Formel V

$H_2NOR_3 \cdot HCl$ (V)

worin $R_3$ die im Anspruch 1 gegebene Bedeutung hat, zum Oximäther der Foreml Ib umsetzt,

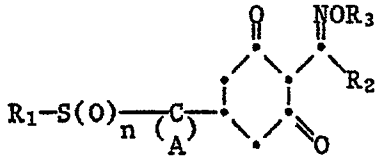

(Ib)

worin A, n, $R_1$, $R_2$ und $R_3$ die im Anspruch 1 gegebene Bedeutung haben.

25. Verfahren zur Herstellung der Acyl-Cyclohexandione und deren Oximäther der Formel I, gemäss Anspruch 1, dadurch gekennzeichnet, dass man in einem inerten organischen Lösungs- oder Verdünnungsmittel, in Gegenwart von Zinkchlorid und einer Stickstoffbase, ein 1,3-Cyclohexandionderivat der Formel II

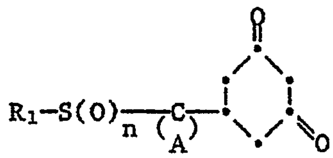

(II)

worin A, n und $R_1$ die im Anspruch 1 gegebene Bedeutung haben, mit einem Säurecyanid der Formel VI umsetzt

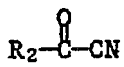

(VI)

worin $R_2$ die im Anspruch 1 gegebene Bedeutung hat, und das erhaltene 2-Acyl-1,3-cyclohexandionderivat der Formel Ia

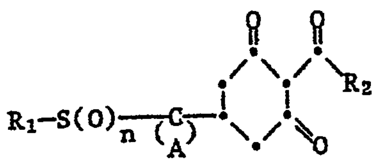

(Ia)

worin A, n, $R_1$ und $R_2$ die im Anspruch 1 gegebene Bedeutung haben, gewünschtenfalls in einem inerten organischen Lösungsmittel, in Gegenwart der äquimolaren Menge einer Base mit einem Hydroxylamin • Hydrochlorid der Formel V

$H_2NOR_3 \cdot HCl$ (V)

worin $R_3$ die im Anspruch 1 gegebene Bedeutung hat, zum Oximäther der Formel Ib umsetzt

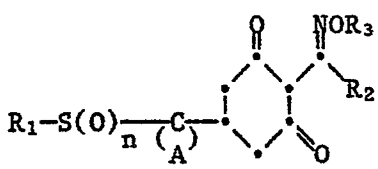

(Ib)

worin A, n, $R_1$, $R_2$ und $R_3$ die im Anspruch 1 gegebene Bedeutung haben.

26. Ein herbizides und den Pflanzenwuchs regulierendes Mittel, dadurch gekennzeichnet, dass es neben Träger- und/oder anderen Zuschlagstoffen als Wirkstoff ein Acyl-Cyclohexandion oder einen Oximäther der Formel I, gemäss Anspruch 1, enthält.

27. Verfahren zur selektiven pre- oder postemergenten Bekämpfung von Ungräsern und Unkräutern in Nutzpflanzenkulturen, dadurch gekennzeichnet, dass man die Nutzpflanzen oder deren Anbaufläche mit einer wirksamen Menge eines Acyl-Cyclohexandiones oder eines Oximäthers der Formel I, gemäss Anspruch 1, oder mit einem, ein solches Derivat enthaltenden Mittels behandelt.

28. Verfahren zur Regulierung des Pflanzenwuchses, dadurch gekennzeichnet, dass man Pflanzen, Pflanzenteile oder Samen mit einer wirksamen Menge eines Acyl-Cyclohexandiones oder eines Oximäthers der Formel I, gemäss Anspruch 1, oder mit einem ein solches Derivat enthaltenden Mittels behandelt.

29. Ein Cyclohexanon-Ester der Formel IV als Zwischenprodukt

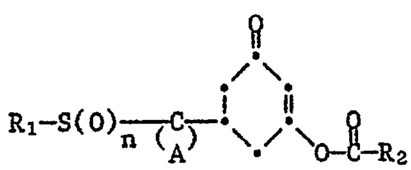

(IV)

worin A, n, $R_1$ und $R_2$ die im Anspruch 1 gegebene Bedeutung haben.

30. Ein Cyclohexanon-Ester der Formel IV gemäss Anspruch 29 worin A die Aethylenbrücke, n Null, $R_1$ Methyl oder Aethyl und $R_2$ Aethyl oder n-Propyl bedeuten.

31. Verfahren zur Herstellung der Cyclohexanon-Ester der Formel IV gemäss Anspruch 30, dadurch gekennzeichnet, dass man ein 1,3-Cyclohexanonderivat der Formel XI

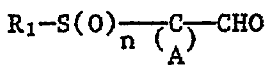 (XI)

worin A, n und $R_1$ die im Anspruch 1 gegebene Bedeutung haben, in einem basischen Lösungsmittel mit Malonsäure zur ungesättigten Säure der Formel XII kondensiert

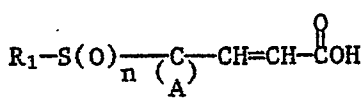 (XII)

worin A, n und $R_1$ die im Anspruch 1 gegebene Bedeutung haben, die Säure dann mit einem Alkohol der Formel XIII verestert,

R—OH (XIII)

worin R $C_1$-$C_6$-Alkyl oder Benzyl bedeutet, den entstandenen Ester der Formel XIV

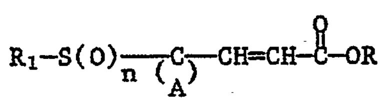 (XIV)

worin A, n und $R_1$ die im Anspruch 1 und R die oben erwähnte Bedeutung haben mit der äquimolaren Menge eines Acetessigsäureester der Formel XV

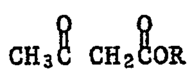 (XV)

worin R die oben gegebene Bedeutung hat, in einem absoluten organischen Lösungsmittel in Gegenwart von Natriummethylat ringgeschlossen zum 1,3-Cyclohexanonderivat der Formel II

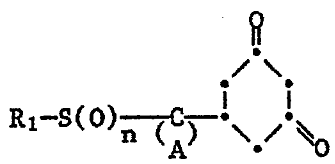 (II)

worin A, n und $R_1$ die im Anspruch 1 gegebene Bedeutung haben, dann in einem inerten organischen Lösungsmittel, in Gegenwart der äquimolaren Menge einer Base mit einem Säurehalogenid oder Säureanhydrid der Formel III umsetzt,

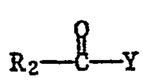 (III)

worin Y ein Halogenatom oder den Rest

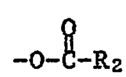

bedeutet und $R_2$ die im Anspruch 1 gegebene Bedeutung hat.

32. 1,3-Cyclohexandionderivate der Formel II als neue Zwischenprodukte

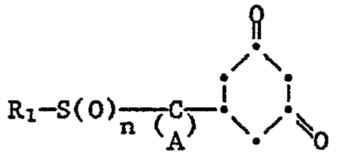

(II)

worin A, n und $R_1$ die im Anspruch 1 gegebene Bedeutung haben.

33. 1,3-Cyclohexanonderivate der Formel II gemäss Anspruch 32 worin A die Aethylbrücke, n Null and $R_1$ Methyl oder Aethyl bedeuten.

34. Verfahren zur Herstellung der 1,3-Cyclohexandionderivate der Formel II

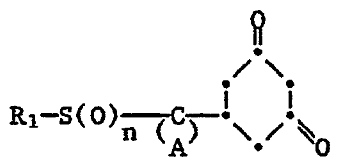

(II)

worin A, n und $R_1$ die im Anspruch 1 gegebene Bedeutung haben, dadurch gekennzeichnet, dass man ein ungesättigtes Methylketon der Formel VII

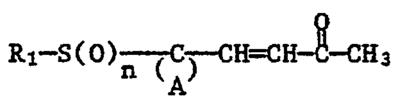

(VII)

worin A, n und $R_1$ die im Anspruch 1 gegebene Bedeutung haben, in einem absoluten, inerten organischen Lösungsmittel in Gegenwart von Alkalimetall-methylat bei Rückflusstemperatur des Lösungsmittels mit einem Malonsäurediester der Formel VIII umgesetzt

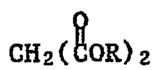

(VIII)

wobei R einem $C_1$-$C_6$-Alkylrest oder Benzyl bedeutet, zum Cyclohex-1-en-2-ol-4-on-ester der Formel IX

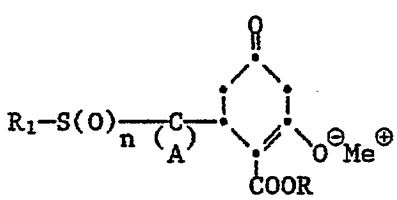

(IX)

worin A, n und $R_1$ die im Anspruch 1 gegebene Bedeutung haben, R einen $C_1$-$C_6$-Alkylrest oder Benzyl und $Me^{\oplus}$ ein Alkalimetallion bedeutet, diesen Ester in Gegenwart von Natron- oder Kalilauge verseift und mit Säure wäscht, das erhaltene 2,4-Cyclohexandionsäure-derivat der Formel X

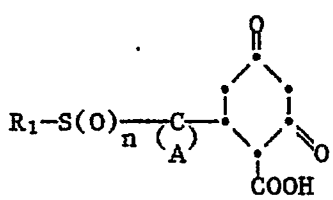

(X)

worin A, n und $R_1$ die im Anspruch 1 gegebene Bedeutung haben, dann in einem inerten Lösungsmittel decarboxyliert und das gewünschte Cyclohexandionderivat der Formel II aus dem Reaktionsgemisch isoliert.

35. Ungesättigte Methylketone der Formel VII als neue Zwischenprodukte

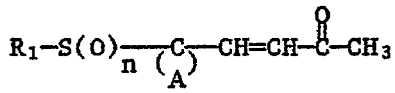

(VII)

worin A, n und $R_1$ die im Anspruch 1 gegebene Bedeutung haben.

36. Ungesättigte Methylketone der Formel VII gemäss Anspruch 35 worin A die Aethylenbrücke, n Null und $R_1$ Methyl oder Aethyl bedeuten.

37. Verfahren zur Herstellung der ungesättigten Methylketone der Formel VII gemäss Anspruch 35, dadurch gekennzeichnet, dass man einen Aldehyd der Formel XI

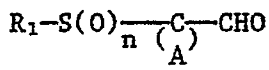

(XI)

worin A, n und $R_1$ die im Anspruch 1 gegebene Bedeutung haben, in basisch wässrigem Milieu mit Aceton kondensiert und das β-Hydroxyketon der Formel XVI

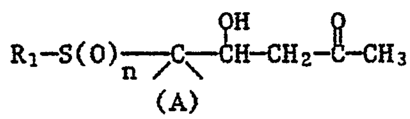

(XVI)

worin A, n und $R_1$ die im Anspruch 1 gegebene Bedeutung haben während mehrer Stunden am Rückfluss kocht und das ungesättigte Keton anschliessend aus dem Reaktionsgemisch isoliert.

## Claims

1. A novel 2-acyl-1,3-cyclohexanedione, or an oxime ether thereof, of formula I

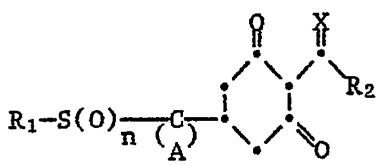

(I).

wherein
A is a 2- to 7-membered alkylene bridge, or a 3- to 7-membered alkenylene bridge which may be mono- or polyunsaturated
n is zero, one or two,
$R_1$ is $C_1$–$C_4$alkyl or benzyl,
$R_2$ is $C_1$–$C_6$alkyl which is unsubstituted or substituted by halogen, $C_1$–$C_4$alkoxy or $C_1$–$C_4$alkylthio; or is $C_3$–$C_6$cycloalkyl; or phenyl, benzyl or phenylethyl, the phenyl ring of each of which may be substituted by halogen, $C_1$–$C_4$haloalkoxy, cyano or nitro,
X is oxygen or a radical $-NOR_3$, and
$R_3$ is $C_1$–$C_6$alkyl, $C_1$–$C_6$halogalkyl, $C_3$-$C_6$alkenyl, $C_3$–$C_6$haloalkenyl or $C_3$–$C_6$alkynyl,
and the salts of these compounds with metals and nitrogen bases.

2. An acylcyclohexanedione according to claim 1 of formula Ia

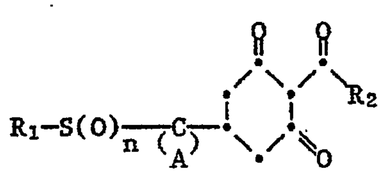

(Ia)

wherein
A is a 2- to 7-membered alkylene bridge,
n is zero, one or two,
$R_1$ is $C_1$–$C_4$alkyl or benzyl and
$R_2$ is $C_1$–$C_6$alkyl which is unsubstituted or substituted by halogen, $C_1$–$C_4$alkoxy or $C_1$–$C_4$alkylthio; or is $C_3$-$C_6$cycloalkyl; or phenyl, benzyl or phenylethyl, the phenyl ring of each of which may be substituted by halogen, $C_1$–$C_4$alkyl, $C_1$–$C_4$alkoxy, $C_1$–$C_4$alkoxy, $C_1$–$C_4$alkylthio, $C_1$–$C_4$haloalkyl, $C_1$–$C_4$haloalkoxy, cyano or nitro.

3. An acylcyclohexanedione according to claim 2 of formula Ia, wherein A is the ethylene bridge, n is zero, $R_1$ is methyl or ethyl and $R_2$ is ethyl or n-propyl.

4. 5-(1-Methylthiocyclobutan-1-yl)-2-(2,4-dichlorobenzoyl)cyclohexane-1,3-dione according to claim 2.

5. 5-(1-Methylthiocyclobutan-1-yl)-2-n-butyrylcyclohexane-1,3-dione according to claim 2.

6. 5-(1-Methylthiocyclobutan-1-yl)-2-cyclopropylcarbonylcyclohexane-1,3-dione according to claim 2.

7. 5-(1-Methylthiocyclobutan-1-yl)-2-(2,3-dichlorobenzoyl)cyclohexane-1,3-dione according to claim 2.

8. 5-(1-Methylsulfonylcyclobutan-1-yl)-2-n-butyrylcyclohexane-1,3-dione according to claim 2.

9. 5-(1-Methylthiocyclopropan-1-yl)-2-n-butyrylcyclohexane-1,3-dione according to claim 2.

10. 5-(1-Methylthiocyclopropan-1-yl)-2-propionylcyclohexane-1,3-dione according to claim 2.

11. 5-(1-Ethylthiocyclopropan-1-yl)-2-propionylcyclohexane-1,3-dione according to claim 2.

12. An acylcyclohexanedione oxime ether according to claim 1 of formula Ib

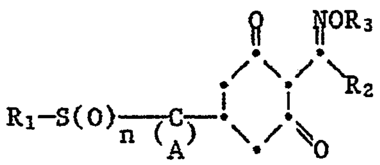

(Ib)

wherein
A is a 2- to 7-membered alkylene bridge,
n is zero, one or two,
$R_1$ is $C_1$–$C_4$alkyl or benzyl,
$R_2$ is $C_1$–$C_6$alkyl which is unsubstituted or substituted by halogen, $C_1$–$C_4$alkoxy or $C_1$–$C_4$alkylthio, and
$R_3$ is $C_1$–$C_6$alkyl, $C_3$–$C_6$haloalkyl, $C_3$–$C_6$alkenyl, $C_3$–$C_6$haloalkenyl or $C_3$–$C_6$alkynyl.

13. An acylcyclohexane oxime ether according to claim 12 of formula Ib, wherein A is the ethylene bridge, n is zero, $R_1$ is methyl or ethyl, $R_2$ is ethyl or n-propyl and $R_3$ is cis- or trans-3-chloroallyl.

14. 5-(1-Methylthiocyclopentan-1-yl)-2-(1-ethoximino-n-butyryl)-cyclohexane-1,3-dione according to claim 12.

15. 5-(1-Methylthiocyclobutan-1-yl)-2-(1-ethoximino-n-butyryl)-cyclohexane-1,3-dione according to claim 12.

16. 5-(1-Methylthiocyclohexan-1-yl)-2-(1-ethoximino-n-butyryl)-cyclohexane-1,3-dione according to claim 12.

17. 5-(1-Methylthiocyclobutan-1-yl)-2-(1-allyloxyimino-n-butyryl)-cyclohexane-1,3-dione according to claim 12.

18. 5-(1-Methylthiocyclopropan-1-yl)-2-(1-ethoximino-n-butyryl)-cyclohexane-1,3-dione according to claim 12.

19. 5-(1-Methylthiocyclopropan-1-yl)-2-[1-(trans-3-chloroallyloximino)-n-butyryl]cyclohexane-1,3-dione according to claim 12.

20. 5-(1-Methylthiocyclopropan-1-yl)-2-[1-(trans-3-chloroallyl-oximino)propionyl]cyclohexane-1,3-dione according to claim 12.

21. 5-(1-Methylthiocyclopropan-1-yl)-2-[1-(cis-3-chloroallyloximino)-propionyl]cyclohexane-1,3-dione according to claim 12.

22. 5-(1-Ethylthiocyclopropan-1-yl)-2-[1-(trans-3-chloroallyloximino)-propionyl]cyclohexane-1,3-dione according to claim 12.

23. 5-(1-Ethylthiocyclopropan-1-yl)-2-[1-(trans-3-chloroallyloximino)-n-butyryl]cyclohexane-1,3-dione according to claim 12.

24. A process for the preparation of an acylcyclohexanedione, or of an oxime ether thereof, of formula I according to claim 1, which process comprises rearranging a 1,3-cyclohexanedione derivative of formula II

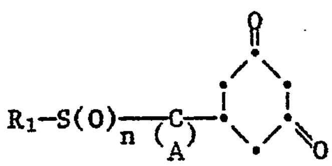

(II)

wherein A, n and $R_1$ are as defined in claim 1, with an acid halide or acid anhydride of formula III

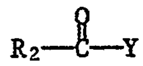 (III)

wherein Y is a halogen atom or a radical

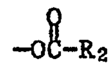

and $R_2$ is as defined in claim 1, in an inert organic solvent and in the presence of the equimolar amount of a base, and rearranging the resultant cyclohexanone ester of formula IV

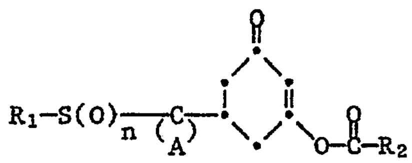 (IV)

wherein A, n, $R_1$ and $R_2$ are as defined in claim 1, in an inert organic solvent and in the presence of a catalyst, to give the 2-acyl-1,3-cyclohexanedione derivative of formula (Ia)

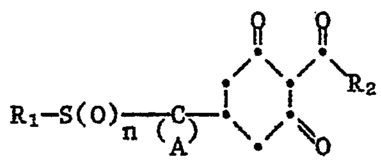 (Ia)

wherein A, n, $R_1$ and $R_2$ are as defined in claim 1, and, if desired, reacting this with a hydroxylamine • hydrochloride of formula V

$H_2NOR_3$ • HCl (V)

wherein $R_3$ is as defined in claim 1, in an inert organic solvent and in the presence of the equimolar amount of a base, to give the oxime ether of formula Ib

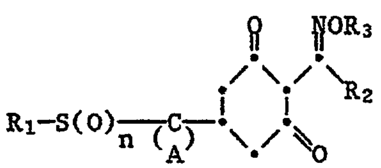 (Ib)

wherein A, n, $R_1$, $R_2$ and $R_3$ are as defined in claim 1.

25. A process for the preparation of an acylcyclohexanedione, or of an oxime ether thereof, of formula I according to claim 1, which process comprises reacting a 1,3-cyclohexanedione derivative of formula II

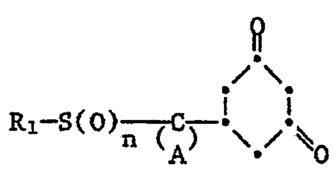 (II)

wherein A, n and $R_1$ are as defined in claim 1, with an acid cyanide of formula VI

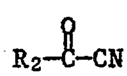 (VI)

wherein $R_2$ is as defined in claim 1, in an inert organic solvent or diluent and in the presence of zinc chloride and a nitrogen base, and reacting the resultant 2-acyl-1,3-cyclohexanedione derivative of formula Ia

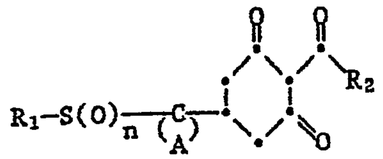

(Ia)

wherein A, n, $R_1$ and $R_2$ are as defined in claim 1, with a hydroxylamine • hydrochloride of formula V
$H_2NOR_3 \bullet HCl$ (V)
wherein $R_3$ is as defined in claim 1, if desired in an inert organic solvent, in the presence of the equimolar amount of a base, to give the oxime ether of formula Ib

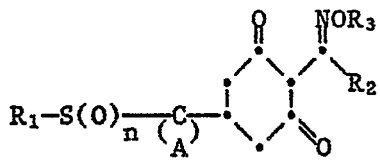

(Ib)

wherein A, n, $R_1$, $R_2$ and $R_3$ are as defined in claim 1.

26. A herbicidal and plant growth regulating agent which contains, as active ingredient, an acylcyclohexanedione, or an oxime ether, of formula I according to claim 1, together with carriers and/or other adjuvants.

27. A method of selectively controlling weeds pre- or postemergence in crops of useful plants, which method comprises treating the useful plants or the crop area thereof with an effective amount of an acylcylohexanedione, or of an oxime ether of formula I according to claim 1, or with an agent which contains such a derivative.

28. A method of regulating plant growth, which method comprises treating plants, parts of plants or seeds with an effective amount of an acylcyclohexanedione, or an oxime ether of formula I according to claim 1, or with an agent which contains such a derivative.

29. A cyclohexanone ester of formula IV as intermediate

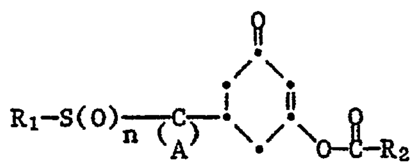

(IV)

wherein A, n, $R_1$ and $R_2$ are as defined in claim 1.

30. A cyclohexanone ester of formula IV according to claim 29, wherein A is the ethylene bridge, n is zero, $R_1$ is methyl or ethyl and $R_2$ is ethyl or n-propyl.

31. A process for the preparation of a cyclohexanone ester of formula IV according to claim 30, which process comprises condensing a 1,3-cyclohexanone derivative of formula XI

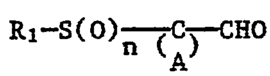

(XI)

wherein A, n and $R_1$ are as defined in claim 1, with malonic acid, in a basic solvent, to give the unsaturated acid of formula XII

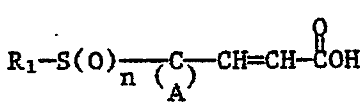

(XII)

wherein A, n and $R_1$ are as defined in claim 1, then esterifying the acid with an alcohol of formula XIII

$R—OH$ (XIII)

wherein R is $C_1$–$C_6$alkyl or benzyl, and ring-closing the resultant ester of formula XIV

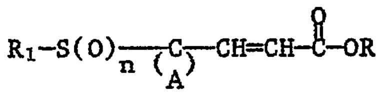 (XIV)

wherein A, n and $R_1$ are as defined in claim 1 and R is as defined above, with the equimolar amount of an acetoacetic acid ester of formula XV

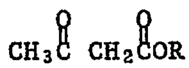 (XV)

wherein R is as defined above, in an absolute organic solvent and in the presence of sodium methylate, to give the 1,3-cyclohexanone derivative of formula II

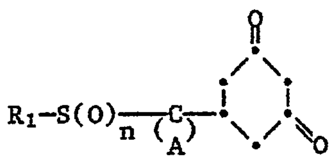 (II)

wherein A, n and $R_1$ are as defined in claim 1, then reacting said derivative of formula II with an acid halide or acid anhydride of formula III

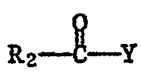 (III)

wherein Y is a halogen atom or the radical

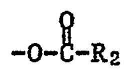

and $R_2$ is as defined in claim 1, in an inert organic solvent and in the presence of the equimolar amount of a base.

32. A 1,3-cyclohexanedione derivative of formula II as novel intermediate

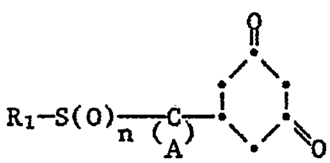 (II)

wherein A, n and $R_1$ are as defined in claim 1.

33. A 1,3-cyclohexanone derivative of formula II according to claim 32, wherein A is the ethyl bridge, n is zero and $R_1$ is methyl or ethyl.

34. A process for the preparation of a 1,3-cyclohexanedione derivative of formula II

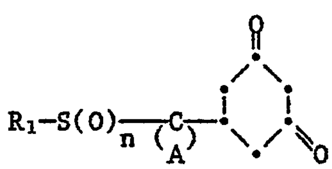 (II)

wherein A, n and $R_1$ are as defined in claim 1, which process comprises reacting an unsaturated methyl ketone of formula VII

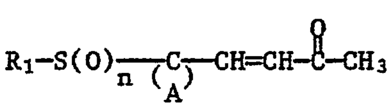 (VII)

wherein A, n and $R_1$ are as defined in claim 1, with a malonic acid diester of formula VIII

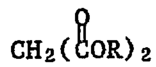 (VIII)

where R is a $C_1$–$C_6$alkyl radical or benzyl, in an absolute inert organic solvent, in the presence of alkali metal methylate and at the reflux temperature of the solvent, to give the cyclohex-1-en-2-ol-4-one ester of formula IX

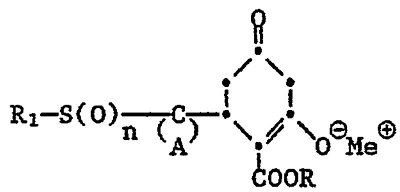 (IX)

wherein A, n and $R_1$ are as defined in claim 1, R is a $C_1$–$C_6$alkyl radical or benzyl and $Me^{\oplus}$ is an alkali metal ion, saponifying this ester in the presence of sodium hydroxide solution or potassium hydroxide solution, washing the saponified ester with acid, then decarboxylating the resultant 2,4-cyclohexanedione acid derivative of formula X

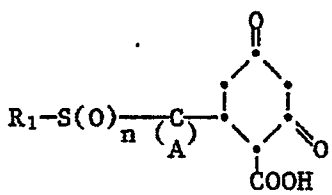 (X)

wherein A, n and $R_1$ are as defined in claim 1, in an inert solvent, and isolating from the reaction mixture the desired cyclohexanedione derivative of formula II.

35. An unsaturated methyl ketone of formula VII as novel intermediate

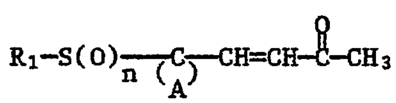 (VII)

wherein A, n and $R_1$ are as defined in claim 1.

36. An unsaturated methyl ketone of formula VII according to claim 35, wherein A is the ethylene bridge, n is zero and $R_1$ is methyl or ethyl.

37. A process for the preparation of an unsaturated methyl ketone of formula VII according to claim 35, which process comprises condensing an aldehyde of formula XI

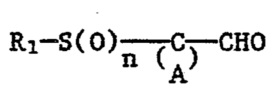 (XI)

wherein A, n and $R_1$ are as defined in claim 1, with acetone, in basic aqueous medium, then boiling the β-hydroxyketone of formula XVI

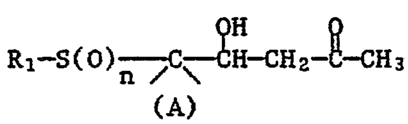 (XVI)

wherein A, n and $R_1$ are as defined in claim 1, for several hours at reflux and subsequently isolating from the reaction mixture the unsaturated ketone.

## Revendications

1. Nouvelles 2-acyl-1,3-cyclohexane-diones et leurs éthers d'oximes de formule I.

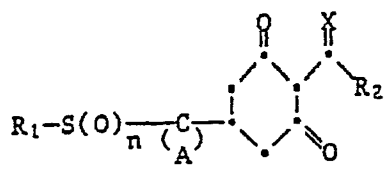

(I)

dans laquelle
A représente un pont alkylène de 2 à 7 chaînons, un pont alcénylène de 3 à 7 chaînons, qui peut être mono- ou poly-insaturé,
n est égal à 0, 1 ou 2,
$R_1$ représente un groupe alkyle en $C_1$–$C_4$ ou benzyle,
$R_2$ représente un groupe alkyle en $C_1$–$C_6$ non substitué ou substitué par des halogènes, des groupes alcoxy en $C_1$–$C_4$ ou alkylthio en $C_1$–$C_4$; un groupe cycloalkyle en $C_3$–$C_6$; un groupe phényle, benzyle ou phényléthyle dans lesquels le noyau phényle peut être substitué par des halogènes, des groupes alkyle en $C_1$–$C_4$, alcoxy en $C_1$–$C_4$, alkoxy en $C_1$–$C_4$, halogénoalkyle en $C_1$–$C_4$, halogénoalcoxy en $C_1$–$C_4$, cyano ou nitro,
X représente l'oxygène ou un groupe $-NOR_3$, et
$R_3$ représente un groupe alkyle en $C_1$–$C_6$, halogénoalkyle en $C_1$–$C_6$, alcényle en $C_3$–$C_6$, halogénoalcényle en $C_3$–$C_6$ ou alcynyle en $C_3$–$C_6$,
ainsi que les sels de ces composés et de métaux ou de bases azotées.

2. Acylcyclohexane-diones selon la revendication 1, de formule Ia

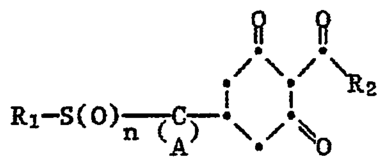

(Ia)

dans laquelle
A représente un pont alkylène de 2 à 7 chaînons,
n est égal à 0, 1 ou 2,
$R_1$ représente un groupe alkyle en $C_1$–$C_4$ ou benzyle, et
$R_2$ représente un groupe alkyle en $C_1$–$C_6$ non substitué ou substitué par des halogènes, des groupes alkyle en $C_1$–$C_4$ ou alkylthio en $C_1$–$C_4$; ou groupe cycloalkyle en $C_3$–$C_6$; un groupe phényle, benzyle ou phényléthyle dans lesquels le noyau phényle peut être substitué par des halogènes, des groupes alkyle en $C_1$–$C_4$, alcoxy en $C_1$–$C_4$, alkylthio en $C_1$–$C_4$, halogénoalkyle en $C_1$–$C_4$, halogénoalcoxy en $C_1$–$C_4$, cyano ou nitro.

3. Acylcyclohexane-diones selon la revendication 2, de formule Ia dans laquelle A représente un pont éthylène, n est éagl à 0, $R_1$ représente un groupe méthyle ou éthyle et $R_2$ un groupe éthyle ou n-propyle.

4. La 5-(1-méthylthiocyclobutane-1-yl)-2-(2,4-dichlorobenzoyl)-cyclohexane-1,3-dione selon la revendication 2.

5. La 5-(1-méthylthiocyclobutane-1-yl)-2-n-butyryl-cyclohexane-1,3-dione selon la revendication 2.

6. La 5-(1-méthylthiocyclobutane-1-yl)-2-cyclopropylcarbonyl-cyclohexane-1,3-dione selon la revendication 2.

7. La 5-(1-méthylthiocyclobutane-1-yl)-2-(2,3-dichlorobenzoyl)-cyclohexane-1,3-dione selon la revendication 2.

8. La 5-(1-méthylsulfonylcyclobutane-1-yl)-2-n-butyryl-cyclohexane-1,3-dione selon la revendication 2.

9. La 5-(1-méthylthiocyclopropane-1-yl)-2-n-butyryl-cyclohexane-1,3-dione selon la revendication 2.

10. La 5-(1-méthylthiocyclopropane-1-yl)-2-propionyl-cyclohexane-1,3-dione selon la revendication 2.

11. La 5-(1-éthylthiocyclopropane-1-yl)-2-propionyl-cyclohexane-1,3-dione selon la revendication 2.

12. Ethers d'oximes d'acyl-cyclohexane-diones selon la revendication 1, de formule Ib

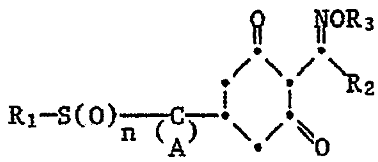 (Ib)

dans laquelle
A représente un pont alkylène de 2 à 7 chaînons,
n est égal à 0, 1 ou 2,
$R_1$ représente un groupe alkyle en $C_1$–$C_4$ ou benzyle,
$R_2$ représente un groupe alkyle en $C_1$–$C_6$ non substitué ou substitué par des halogènes, des groupes alcoxy en $C_1$–$C_4$ ou alkylthio en $C_1$–$C_4$,
$R_3$ représente un groupe alkyle en C 1–$C_6$, halogénoalkyle en $C_3$–$C_6$, alcényle en $C_3$–$C_6$, halogénoalcényle en $C_3$–$C_6$ ou alcynyle en $C_3$–$C_6$.

13. Ethers d'oximes d'acylcyclohexane-diones selon la revendication 12, de formule Ib dans laquelle A représente le pont éthylène, n est égal à 0, $R_1$ représente un groupe méthyle ou éthyle, $R_2$ un groupe éthyle ou n-propyle et $R_3$ un groupe cis- ou trans-3-chlorallyle.

14. La 5-(1-méthylthiocyclopentane-1-yl)-2-(1-éthoximino-n-butyryl)-cyclohexane-1,3-dione selon la revendication 12.

15. La 5-(1-méthylthiocyclobutane-1-yl)-2-(1-éthoximino-n-butyryl)-cyclohexane-1,3-dione selon la revendication 12.

16. La 5-(1-méthylthiocyclohexane-1-yl)-2-(1-éthoximino-n-butyryl)-cyclohexane-1,3-dione selon la revendication 12.

17. La 5-(1-méthylthiocyclobutane-1-yl)-2-(1-allyloxyimino-n-butyryl)-cyclohexane-1,3-dione selon la revendication 12.

18. La 5-(1-méthylthiocyclopropane-1-yl)-2-(1-éthoximino-n-butyryl)-cyclohexane-1,3-dione selon la revendication 12.

19. La 5-(1-méthylthiocyclopropane-1-yl)-2-[1-(trans-3-chlorallyloximino)-n-butyryl]-cyclohexane-1,3-dione selon la revendication 12.

20. La 5-(1-méthylthiocyclopropane-1-yl)-2-[1-(trans-3-chlorallyl-oximino)-propionyl]-cyclohexane-1,3-dione selon la revendication 12.

21. La 5-(1-méthylthiocyclopropane-1-yl)-2-[1-(cis-3-chlorallyl-oximino)-propionyl]-cyclohexane-1,3-dione selon la revendication 12.

22. La 5-(1-éthylthiocyclopropane-1-yl)-2-[1-(trans-3-chlorallyl-oximino)-propionyl]-cyclohexane-1,3-dione selon la revendication 12.

23. La 5-(1-éthylthiocyclopropane-1-yl)-2-[1-(trans-3-chlorallyl-oximino)-n-butyryl]-cyclohexane-1,3-dione selon la revendication 12.

24. Procédé de préparation des acyl-cyclohexane-diones et de leurs éthers d'oximes de formule I selon la revendication 1, caractérisé en ce que l'on fait réagir dans un solvant organique inerte, en présence de la quantité équimoléculaire d'une base, un dérivé de 1,3-cyclohexane-dione de formule II

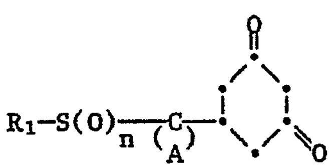 (II)

dans laquelle A, n et $R_1$ ont les significations indiquées dans la revendication 1, avec un halogénure d'acide ou un anhydride de formule III

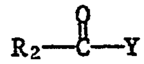 (III)

dans laquelle Y représente un atome d'halogène ou un groupe

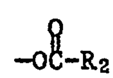

et $R_2$ a les significations indiquées dans la revendication 1, ce qui donne un cyclohexanone-ester de formule IV

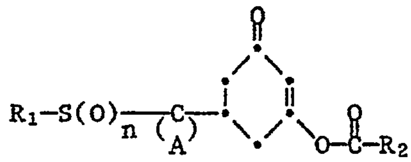 (IV)

dans laquelle A, n, $R_1$ et $R_2$ ont les significations indiquées dans la revendication 1, qu'on transpose dans un solvant organique, en présence d'un catalyseur, en le dérivé de 2-acyl-1,3-cyclohexane-dione de formule Ia

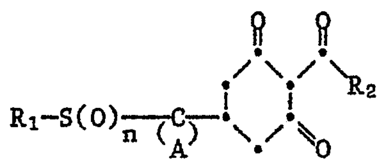 (Ia)

dans laquelle A, n, $R_1$ et $R_2$ ont les significations indiquées dans la revendication 1, qu'on convertit si on le désire, dans un solvant organique inerte, en présence de la quantité équimoléculaire d'une base, par réaction avec un chlorhydrate d'hydroxylamine de formule V
$H_2NOR_3 \bullet HCl$ (V)
dans laquelle $R_3$ a les significations indiquées dans la revendication 1, en l'éther d'oxime de formule Ib

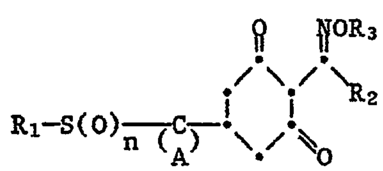 (Ib)

dans laquelle A, n, $R_1$, $R_2$ et $R_3$ ont les significations indiquées dans la revendication 1.

25. Procédé de préparation des acyl-cyclohexane-diones et de leurs éthers d'oximes de formule I selon la revendication 1, caractérisé en ce que l'on fait réagir en présence du chlorure de zinc et d'une base azotée un dérivé de 1,3-cyclohexane-dione de formule II

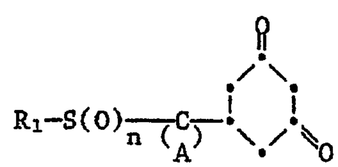 (II)

dans laquelle A, n et $R_1$ ont les significations indiquées dans la revendication 1, avec un cyanure d'acide de formule VI

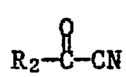 (VI)

dans laquelle $R_2$ a les significations indiquées dans la revendication 1, ce qui donne un dérivé de 2-acyl-1,3-cyclohexane-dione de formule Ia

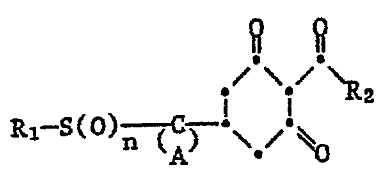 (Ia)

dans laquelle A, n, $R_1$ et $R_2$ ont les significations indiquées dans la revendication 1, qu'on convertit si on le désire, dans un solvant organique inerte, en présence de la quantité équimoléculaire d'une base, par réaction avec un chlorhydrate d'hydroxylamine de formule V
$H_2NOR_3 \bullet HCl$ (V)

dans laquelle $R_3$ a les significations indiquées dans la revendication 1, en l'éther d'oxime de formule Ib

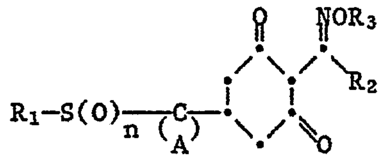

(Ib)

dans laquelle A, n, $R_1$, $R_2$ et $R_3$ ont les significations indiquées dans la revendication 1.

26. Un produit herbicide et régulateur de la croissance des végétaux, caractérisé en ce qu'il contient en tant que substance active, avec des véhicules et/ou d'autres additifs, une acyl-cyclohexane-dione ou un éther d'oxime de formule I selon la revendication 1.

27. Procédé pour combattre sélectivement en pré-levée ou en post-levée les mauvaises herbes et les végétaux adventices dans les cultures de végétaux utiles, caractérisé en ce que l'on traite les plantes utiles ou leur aire de culture par une quantité efficace d'une acyl-cyclohexane-dione ou d'un éther d'oxime de formule I selon la revendication 1 ou d'un produit contenant un tel dérivé.

28. Procédé pour la régulation de la croissance des végétaux, caractérisé en ce que l'on traite les plantes, des parties des plantes ou les semences par une quantité efficace d'une acyl-cyclohexane-dione ou d'un éther d'oxime de formule I selon la revendication 1 ou d'un produit contenant un tel dérivé.

29. Un cyclohexane-ester de formule IV

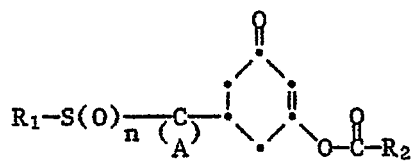

(IV)

dans laquelle A, n, $R_1$ et $R_2$ ont les significations indiquées dans la revendication 1, en tant que produit intermédiaire.

30. Un cyclohexanone-ester de formule IV selon la revendication 29, dans laquelle A représente le pont éthylène, n est égal à 0, $R_1$ représente un groupe méthyle ou éthyle et $R_2$ un groupe éthyle ou n-propyle.

31. Procédé de préparation des cyclohexanone-esters de formule IV selon la revendication 30, caractérisé en ce que l'on condense un dérivé de 1,3-cyclohexanone de formule XI

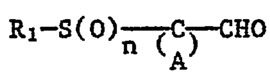

(XI)

dans laquelle A, n et $R_1$ ont les significations indiquées dans la revendication 1, dans un solvant basique, avec l'acide malonique, ce qui donne l'acide insaturé de formule XII

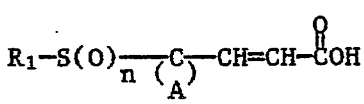

(XII)

dans laquelle A, n et $R_1$ ont les significations indiquées dans la revendication 1, qu'on estérifie par un alcool de formule XIII
R–OH XIII
dans laquelle R représente un groupe alkyle en $C_1$–$C_6$ ou benzyle, ce qui donne l'ester de formule XIV

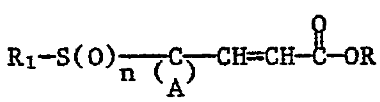

(XIV)

dans laquelle A, n et $R_1$ ont les significations indiquées dans la revendication 1, et R les significations indiquées ci-dessus, qu'on cyclise par la quantité équimoléculaire d'un ester acétylacétique de formule XV

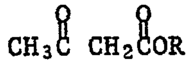

(XV)

dans laquelle R a les significations indiquées ci-dessus, dans un solvant organique anhydre, en présence de méthylate de sodium, en le dérivé de 1,3-cyclohexanone de formule II

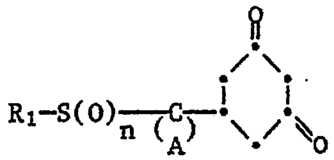

(II)

dans laquelle A, n et $R_1$ ont les significations indiquées dans la revendication 1, qu'on fait réagir dans un solvant organique inerte, en présence de la quantité équimoléculaire d'une base, avec un halogénure d'acide ou un anhydride de formule III

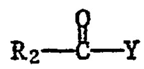

(III)

dans laquelle Y représente un atome d'halogène ou le groupe

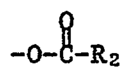

et $R_2$ a les significations indiquées dans la revendication 1.

32. Dérivés de la 1,3-cyclohexane-dione de formule II

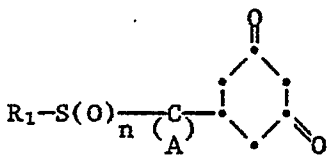

(II)

dans laquelle A, n et $R_1$ ont les significations indiquées dans la revendication 1, en tant que nouveaux produits intermédiaires.

33. Dérivés de la 1,3-cyclohexanone de formule II selon la revendication 32, dans lesquels A représente le pont éthyle, n est égal à 0 et $R_1$ représente un groupe méthyle ou éthyle.

34. Procédé de préparation des dérivés de la 1,3-cyclohexane-dione de formule II

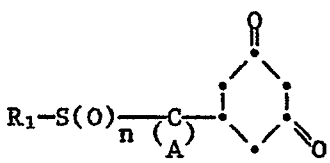

(II)

dans laquelle A, n et $R_1$ ont significations indiquées dans la revendication 1, caractérisé en ce que l'on fait réagir une méthylcétone insaturée de formule VII

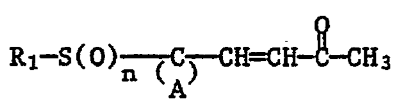

(VII)

dans laquelle A, n et $R_1$ ont les significations indiquées dans la revendication 1, dans un solvant organique inerte anhydre, en présence d'un méthylate de métal alcalin, à la température de reflux du solvant, avec un diester malonique de formule VIII

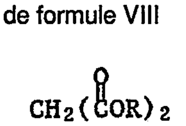

(VIII)

dans laquelle R représente un groupe alkyle en $C_1$–$C_6$ ou benzyle, ce qui donne un cyclohexa-1-ène-2-o1-4-one-ester de formule IX

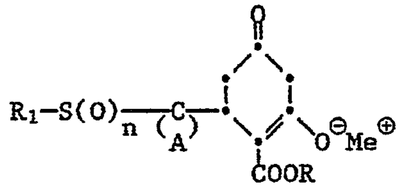

(IX)

dans laquelle A, n et $R_1$ ont les significations indiquées dans la revendication 1, R représente un groupe alkyle en $C_1$–$C_6$ ou benzyle et $Me^{\oplus}$ un ion de métal alcalin, qu'on saponifie en présence de lessive de soude ou de potasse et qu'on lave avec un acide, ce qui donne le dérivé d'acide 2,4-cyclohexane-dione-carboxylique de formule X

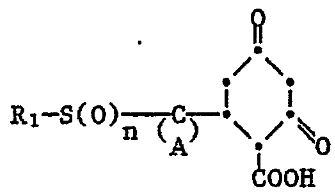

(X)

dans laquelle A, n et $R_1$ ont les significations indiquées dans la revendication 1, qu'on soumet à décarboxylation dans un solvant inerte, après quoi on isole du mélange de réaction le dérivé de cyclohexane-dione de formule II recherché.

35. Méthylcétones insaturées de formule VII

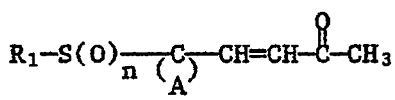

(VII)

dans laquelle A, n et $R_1$ ont les significations indiquées dans la revendication 1, en tant que nouveaux produits intermédiaires.

36. Méthylcétones insaturées de formule VII selon la revendication 35, dans lesquelles A représente le pont éthylène, n est égal à 0 et $R_1$ représente un groupe méthyle ou éthyle.

37. Procédé de préparation des méthylcétones insaturées de formule VII de la revendication 35, caractérisé en ce que l'on condense un aldéhyde de formule XI

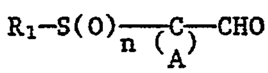

(XI)

dans laquelle A, n et $R_1$ ont les significations indiquées dans la revendication 1, en milieu aqueux basique, avec l'acétone, ce qui donne une bêta-hydroxycétone de formule XVI

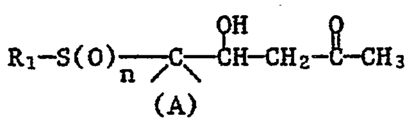

(XVI)

dans laquelle A, n et $R_1$ ont les significations indiquées dans la revendication 1, qu'on fait bouillir au reflux pendant plusieurs heures, après quoi on isole la cétone insaturée du mélange de réaction.